# EUROPEAN PATENT APPLICATION

(11) **EP 4 303 231 A1**
(43) Date of publication of application: **10.01.2024**
(21) Application number: 22749007.5
(22) Date of filing: 27.01.2022
(51) Int. Cl.: C07K 16/00, C07K 16/46, C07K 14/74

(54) **BISPECIFIC ANTIBODY**

(30) Priority: 07.02.2021 CN 202110178932; 16.08.2021 CN 202110937430
(71) Applicant: CHIA TAI TIANQING PHARMACEUTICAL GROUP CO., LTD., Lianyungang, Jiangsu 222062 (CN)
(72) Inventor: WANG, Liangliang, Nanjing City, Jiangsu 211100 (CN); ZHANG, Zhengping, Nanjing City, Jiangsu 211100 (CN); SUN, Congcong, Nanjing City, Jiangsu 211100 (CN); ZHAO, Rongjuan, Nanjing City, Jiangsu 211100 (CN); LI, Yimeng, Nanjing City, Jiangsu 211100 (CN)
(74) Representative: v. Bezold & Partner Patentanwälte - PartG mbB
(86) International application number: PCT/CN2022/074133
(87) International publication number: WO 2022/166728

(57) **Abstract**

Provided is a bispecific antibody, comprising a first antigen-binding moiety and a second antigen-binding moiety, wherein a paired domain of the first antigen-binding moiety comprises an amino acid sequence of engineered HLA-I α3 and an amino acid sequence of engineered β2M. Also provided are an expression method for the bispecific antibody, a polynucleotide encoding the bispecific antibody, a vector and host cell containing the polynucleotide, a pharmaceutical composition containing the bispecific antibody, a fusion protein, and a conjugate. The bispecific antibody provided can prevent or attenuate mismatches between heavy and light chains of different specificities.

## Description

### TECHNICAL FIELD

The present disclosure relates to antibodies, and particularly to a bispecific antibody that improves light and heavy chain mispairing in the antibody.

### BACKGROUND

Nisonoff and colleagues first described the concept of an artificial antibody molecule with two different antigen-binding sites, a bispecific antibody (BsAb), in the early 1960s (Nisonoff et al., Science, 132, 1770-1771 (1960)). They used a slight reoxidation to couple rabbit antigen-binding fragments (Fabs) with different specificities, showing that these two antigen-binding fragments mediate agglutination of two different types of cells (Fudenberg et al., A. J.Exp. Med., 119, 151-166 (1964)). Following a series of conceptual and technological innovations, developed with significant advances in antibody engineering and antibody biology, the bispecific antibody concept is being commercialized by biotechnology and pharmaceutical companies to create novel and unique therapeutic methods.

In the development and design of bispecific antibodies, it is necessary to ensure the correct pairing of two pairs of light chains and heavy chains with different specificities on the basis of the clinical therapeutic purpose. It is also necessary to maintain the independence of the respective binding domains of each monoclonal antibody, so as to ensure that the binding of different epitopes does not generate steric hindrance interference. At the same time, it is also required that the antibody molecules are easily expressed by mammalian cells without complex protein modification processes. Bispecific antibodies in the form of asymmetric molecular design seek to retain the natural structure of natural antibodies as much as possible in order to maintain relevant functional characteristics and favorable quality attributes. The problem of chain mispairing is solved by breaking the symmetry of antibody H2L2 assembly.

Bispecific antibodies have become one of the novel drug research fields in tumor therapy due to the advantage of dual targeting. The construction and preparation technology of bispecific antibodies has also undergone a series of innovations, and one of the purposes is to solve the problem of mispairing, thereby improving the production efficiency. Some bispecific antibodies need to co-express two different heavy chains and two different light chains, and bispecific antibodies of interest also need to be selected from a variety of recombinant products, which has been one of the challenges in the development of bispecific antibodies. To solve the chain mispairing, scientists have developed a number of strategies and technical platforms that can design and express bispecific antibodies with different structures in recent years. At present, there are dozens of bispecific antibody structures, which are derived from different preparation platforms. The more mature ones are Genentech/Roche knob-into-hole, Amgen BiTe, and Chugai/Roche ART-Ig platforms, in which Knob-into-hole is a classic technology for solving the problem of heavy chain mispairing, and there are other technical platforms such as DVD-Ig, TrioMab, DART, FIT-Ig, WuXiBody, and the like.

Most bispecific antibodies in the asymmetric form achieve heterodimeric assembly of two different heavy chains by introducing mutations into the Fc region (Ridgway et al., Protein Engineering, Design and Selection, 9(7), 617-621) that force correction and promote pairing between the heavy chains. The desired final product is then isolated by a differential Protein A binding design purification strategy combined with sequential affinity chromatography and molecular weight differential chromatography purification strategies. To avoid light and heavy chain mispairing strategies, bispecific molecules are usually expressed separately as half molecules or parent antibodies, then the antibody half molecules are assembled, and heavy chain heterodimers can be achieved by designing heavy chain mutations. However, a selective pairing of respective light and heavy chains in individual antibodies remains challenging.

### SUMMARY

The present disclosure provides a bispecific antibody with an improved structural design, which prevents or attenuates the mispairing of heavy and light chains with different specificities.

In one aspect, the present disclosure provides a bispecific antibody comprising a first antigen-binding portion and a second antigen-binding portion that specifically bind to two different antigens or different epitopes of the same antigen, wherein the first antigen-binding portion comprises:
a first polypeptide comprising, from N-terminus to C-terminus, a first heavy chain variable domain and a first paired domain operably linked to the first heavy chain variable domain, and
a second polypeptide comprising, from N-terminus to C-terminus, a first light chain variable domain and a second paired domain operably linked to the first light chain variable domain,
wherein one of the first paired domain and the second paired domain comprises an amino acid sequence of engineered HLA-I α3, and the other paired domain comprises an amino acid sequence of engineered β2 microglobulin.

In some embodiments, the first paired domain and the second paired domain can form a dimer, at least one non-natural interchain bond can be formed between the first paired domain and the second paired domain, and the non-natural interchain bond can stabilize the dimer.

In some embodiments, the amino acid sequence of the engineered HLA-I α3 has at least 85%, 86%, 87%, 88%, 89%, 90%, 91%, 92%, 93%, 94%, 95%, 96%, 97%, 98%, 99%, or 100% identity to a sequence set forth in SEQ ID NO: 1, and the amino acid sequence of the engineered β2 microglobulin has at least 85%, 86%, 87%, 88%, 89%, 90%, 91%, 92%, 93%, 94%, 95%, 96%, 97%, 98%, 99%, or 100% identity to a sequence set forth in SEQ ID NO: 2.

In some specific embodiments, the engineered HLA-I α3 comprises an amino acid sequence set forth in SEQ ID NO: 3, and the engineered β2 microglobulin comprises an amino acid sequence set forth in SEQ ID NO: 4.

In some specific embodiments, the engineered HLA-I α3 comprises an amino acid sequence set forth in SEQ ID NO: 3, and the engineered β2 microglobulin comprises an amino acid sequence set forth in SEQ ID NO: 5.

In some embodiments, the second antigen-binding portion comprises a second heavy chain variable domain and a second light chain variable domain.

In some embodiments, the second antigen-binding portion comprises a Fab comprising the second heavy chain variable domain and the second light chain variable domain.

In some embodiments, provided is a bispecific antibody comprising a first antigen-binding portion and a second antigen-binding portion that specifically bind to two different antigens or different epitopes of the same antigen, wherein the first antigen-binding portion comprises:
a first polypeptide comprising, from N-terminus to C-terminus, a first heavy chain variable domain and a first paired domain operably linked to the first heavy chain variable domain, and
a second polypeptide comprising, from N-terminus to C-terminus, a first light chain variable domain and a second paired domain operably linked to the first light chain variable domain,
wherein one of the first paired domain and the second paired domain comprises an amino acid sequence of engineered HLA-I α3, and the other paired domain comprises an amino acid sequence of engineered β2 microglobulin; the amino acid sequences of the engineered HLA-I α3 and the engineered β2 microglobulin have at least 85%, 86%, 87%, 88%, 89%, 90%, 91%, 92%, 93%, 94%, 95%, 96%, 97%, 98%, 99%, or 100% identity to sequences set forth in SEQ ID NO: 1 and SEQ ID NO: 2, respectively; the second antigen-binding portion comprises a Fab. In some such embodiments, an amino acid in a CL domain of the Fab is substituted with alanine at position F118 according to an EU numbering. In some embodiments, the engineered HLA-I α3 comprises an amino acid sequence set forth in SEQ ID NO: 3, and the engineered β2 microglobulin comprises an amino acid sequence set forth in SEQ ID NO: 4 or SEQ ID NO: 5.

In some embodiments, provided is a bispecific antibody comprising a first antigen-binding portion and a second antigen-binding portion that specifically bind to two different antigens or different epitopes of the same antigen, wherein the first antigen-binding portion comprises:
a first polypeptide comprising, from N-terminus to C-terminus, a first heavy chain variable domain and a first paired domain operably linked to the first heavy chain variable domain, and
a second polypeptide comprising, from N-terminus to C-terminus, a first light chain variable domain and a second paired domain operably linked to the first light chain variable domain,
wherein one of the first paired domain and the second paired domain comprises an amino acid sequence of engineered HLA-I α3, and the other paired domain comprises an amino acid sequence of engineered β2 microglobulin; the second antigen-binding portion comprises a Fab, and an amino acid in a CL domain of the Fab is substituted with alanine at position F118 according to an EU numbering. In some such embodiments, the amino acid sequences of the engineered HLA-I α3 and the engineered β2 microglobulin have at least 85%, 86%, 87%, 88%, 89%, 90%, 91%, 92%, 93%, 94%, 95%, 96%, 97%, 98%, 99%, or 100% identity to sequences set forth in SEQ ID NO: 1 and SEQ ID NO: 2, respectively. Further, in some embodiments, the engineered HLA-I α3 comprises an amino acid sequence set forth in SEQ ID NO: 3, and the engineered β2 microglobulin comprises an amino acid sequence set forth in SEQ ID NO: 4 or SEQ ID NO: 5.

In some of the above embodiments, the first paired domain comprises an amino acid sequence of the engineered HLA-I α3, and the second paired domain comprises an amino acid sequence of the engineered β2 microglobulin.

In some of the above embodiments, the first paired domain comprises an amino acid sequence of the engineered β2 microglobulin, and the second paired domain comprises an amino acid sequence of the engineered HLA-I α3.

In some of the above embodiments, the bispecific antibody comprises an Fc.

In one aspect, the present disclosure provides an isolated polynucleotide encoding the bispecific antibody described herein.

In one aspect, the present disclosure provides an isolated vector comprising the polynucleotide described herein.

In one aspect, the present disclosure provides a host cell comprising the isolated polynucleotide or the isolated vector described herein.

In another aspect, the present disclosure provides a method for expressing the bispecific antibody comprising culturing the host cell under such conditions that the bispecific antibody is expressed.

In another aspect, the present disclosure provides a pharmaceutical composition comprising the bispecific antibody described herein and a pharmaceutically acceptable carrier.

In another aspect, the present disclosure provides a conjugate comprising the bispecific antibody described herein and a therapeutic agent linked or conjugated to the bispecific antibody.

In another aspect, the present disclosure provides a fusion protein comprising a fusion component expressed by fusion and the bispecific antibody.

In yet another aspect, the present disclosure provides a method of treating a disease in a subject in need thereof, comprising administering to the subject a therapeutically effective amount of the bispecific antibody, the pharmaceutical composition, the conjugate, or the fusion protein described herein.

In the bispecific antibody of the present disclosure, the paired domains in the first antigen-binding portion can improve chain mispairing using the engineered HLA-I α3 and the engineered β2 microglobulin. For example, the first antigen-binding portion and the second antigen-binding portion are less likely to occur mispairing compared to the case where both the first and second antigen-binding portions are counterparts of a natural Fab, thereby improving the yield and purity of the antibody of interest.

Particularly, the bispecific antibody is designed to retain good affinity or/and thermal stability to an antigen. In certain aspects, the bispecific antibody is designed to reduce the risk of immunogenicity.

### BRIEF DESCRIPTION OF THE DRAWINGS

FIG. 1 shows a consensus sequence of an α chain constant region of an HLA-I-B molecule;
FIG. 2 is a structural schematic view of anti-CD3/CD38 bispecific antibodies constructed based on MHC-I elements;
FIG. 3 is a structural schematic view of an MHI383-ccff-IgG 1 molecule;
FIG. 4 is an SEC purification map of an MHI383-ccff-IgG 1 molecule;
FIG. 5 is a complete molecular weight mass spectrometry map of MHI383-ccff-IgGl;
FIG. 6 shows a non-reducing SDS-PAGE in which heavy and light chains containing MHCICα, MHCICβ, CH1, and CL domains are paired with each other for expression;
FIG. 7 shows a non-reducing SDS-PAGE of HZ5G11VL-IgK or expression products combined with HZ14A9VH-Cα-IgG1 after introduction of amino acid mutations;
FIG. 8 is a structural schematic view of MHL147-3322-IgG1-wt bispecific antibody constructed based on MHC-I elements;
FIG. 9 is a structural schematic view of MHL147-3322-IgG1-F118A bispecific antibody constructed based on MHC-I elements;
FIG. 10 shows the effect of different concentrations of the bispecific antibodies MHL147-3322-IgG1-wt and MHL147-3322-IgG1-F118A on red blood cell agglutination;
FIG. 11 shows a non-reducing SDS-PAGE of antibodies HZ5G11 and HZ14A9 after introduction of F118A into CLs; and

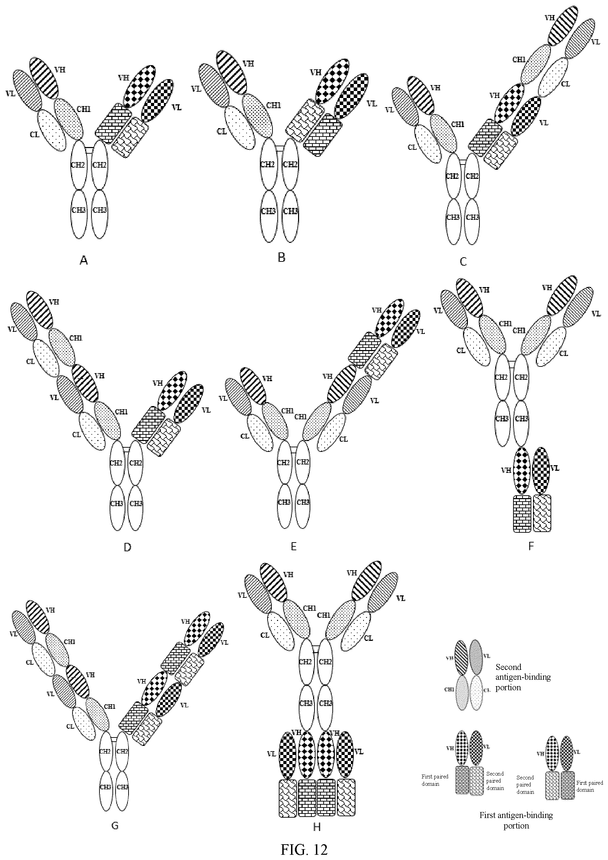

FIG. 12 is a diagram of some exemplary structural forms of bispecific antibodies of the present disclosure, wherein (A) represents "1 + 1" form, in which an MHC-modified antigen-binding portion with different specificities (a first antigen-binding portion) and a Fab are linked to N-terminus of an Fc polypeptide, respectively; (B) represents "1 + 1" form, in which an MHC-modified antigen-binding portion with different specificities (a first antigen-binding portion with Cα and Cβ in different orientations from (A)) and a Fab are linked to an Fc polypeptide, respectively; (C) represents "2 + 1" form, in which a first antigen-binding portion and one Fab are linked to an Fc polypeptide, respectively, and the other Fab is linked to N-terminus of the first antigen-binding portion; (D) represents "2 + 1" form different from (C), in which two Fabs are linked in series; (E) represents "2 + 1" form, in which two Fabs are linked to an Fc polypeptide, respectively, and a first antigen-binding portion is linked to N-terminus of one Fab; (F) represents "2 + 1" form different from (E), in which a first antigen-binding portion is linked to C-terminus of an Fc polypeptide; (G) represents "2 + 2" form, in which antigen-binding portions are linked to N-terminus of an Fc; (H) represents "2 + 2" form, in which first antigen-binding portions are both linked to C-terminus of an Fc; the Fc domain may also have amino acid substitutions such as KIH, and the CL may have F118A, with the amino acid substitutions not shown in this legend.

### Terminology

The term "antibody" is used herein in its broadest sense to refer to a protein comprising an antigen-binding site and encompasses natural and artificial antibodies of various structures, including but not limited to, monoclonal antibodies, monospecific antibodies, multispecific antibodies (e.g., bispecific antibodies, trispecific antibodies, and the like), single-chain antibodies, and the like.

The term "multispecific" means that an antibody can specifically bind to at least two different antigenic determinants. Generally, a bispecific antibody comprises two antigen-binding sites, each of which is specific for a different antigenic determinant. Different antigenic determinants may be expressed on the same or different cells. Different antigenic determinants may be different depending on different types of antigens (e.g., binding to antigens PDL1 and CD47) or may be present on the same antigen. An antigenic determinant is a special chemical group with a certain composition and structure on the surface or other parts of the antigenic substance molecule, which can specifically bind to its corresponding antibody or sensitized lymphocyte. An example of the antigenic determinant is an antigenic determinant CD47, which is an antigenic substance having various structurally defined or structurally undefined antigenic determinants. A specific bispecific antibody can, for example, bind to PDL1 and CD47.

The term "specifically bind to" means that the binding is selective for an antigen and can be distinguished from interactions that are not desired or are non-specific.

The term "...valent" antibody refers to the number of antigen-binding sites present in the antibody. A "bivalent" antibody refers to the presence of two antigen-binding sites in the antibody, and "trivalent" refers to the presence of three antigen-binding sites in the antibody. A natural human immunoglobulin molecule typically has two antigen-binding sites, and a Fab typically has a single antigen-binding site. A single variable domain and an ScFv typically have a single antigen-binding site.

The term "antigen-binding portion" refers to a polypeptide molecule specifically binding to an antigenic determinant. Specific antigen-binding portions may be Fab, ScFv, a single variable domain, or the like. The antigenic determinant is synonymous with an antigen epitope herein.

The term "first", "second", or "third" used herein with respect to an antigen-binding portion, an antigen, a heavy chain variable domain, a light chain variable domain, an Fc polypeptide, and the like are used for ease of distinction when more than one part of each type is present. Unless specifically stated, the use of these terms is not intended to confer a particular order or orientation to the bispecific antibody.

The term "operably link" or "operably linked" refers to the juxtaposition of two or more biological sequences of interest in such a way that they are in a relationship permitting them to function in their intended manner, whether or not a spacer or linker is present. When used with respect to polypeptides, the term is intended to indicate that the polypeptide sequences are linked in such a way that the product of the linkage has the intended biological function. For example, an antibody variable region can be operably linked to a constant region to form a stable product with antigen-binding activity. The term can also be used with respect to polynucleotides. For example, when a polynucleotide encoding a polypeptide is operably linked to a regulatory sequence (e.g., a promoter, enhancer, or silencer sequence), the term is intended to indicate that the polynucleotide sequence is linked in a way that allows for regulated expression of the polypeptide from the polynucleotide.

Major histocompatibility complex (MHC) is a generic term for a group of genes that encode major histocompatibility antigens in animals. Mouse MHC is referred to as H-2 gene complex. Human MHC is referred to as human leukocyte antigen (HLA) gene complex, and its encoded product is referred to as HLA molecule, HLA antigen, or human leukocyte antigen. The HLA gene complex is located at the short arm 6p21.3 of human chromosome 6 and contains more than 220 genes with different functions at the same time. Most of these genes encode proteins in an immune system. MHC class I molecules are expressed on the surface of almost all nucleated cells and are recognized by CD8+ T cells. MHC class II molecules are expressed on the surface of antigen-presenting cells and are recognized by CD4+ T cells. MHC is polymorphic, and the majority of the population mainly comprises 6 classical MHC molecules: 3 classical MHC class I molecules (HLA-A, HLA-B, and HLA-C) and 3 classical MHC class II molecules (HLA-DR, HLA-DP, and HLA-DQ). A human MHC class I molecule (HLA-1) consists of a heavy chain (α chain) and β2 microglobulin (β₂m, or β chain), wherein the extracellular segment of the α chain has three domains (α1, α2, and α3), the 2 domains α1 and α2 at the membrane-distal end form an antigen-binding groove, and the domain α3 is homologous to a human immunoglobulin constant region.

The term "variable domain" or "variable region" refers to a domain of an antibody that is involved in the binding of the antibody to an antigen. For example, a natural four-chain antibody (e.g., derived from human, murine, and the like) has a heavy chain variable region (VH) and a light chain variable region (VL), and a heavy-chain antibody derived from an animal such as camelid or shark has a single variable domain. Each variable domain of a natural antibody consists essentially of four "framework regions" and three "complementarity determining regions". The four framework regions are referred to as framework region 1 (or FR1), framework region 2 (or FR2), framework region 3 (or FR3), and framework region 4 (or FR4), respectively. The framework regions are separated by three complementarity determining regions (or CDRs) referred to in the art and hereinafter as complementarity determining region 1 (or CDR1), complementarity determining region 2 (or CDR2), and complementarity determining region 3 (or CDR3), respectively. Thus, the general structure of a variable domain can be represented as follows: FR1-CDR1-FR2-CDR2-FR3-CDR3-FR4. Variable domains impart specificity for an antigen to an antibody by virtue of having an antigen-binding site.

"CDR" (complementarity determining region) is also referred to as "hypervariable region (HVR)". A natural four-chain antibody typically comprises six CDRs, three in the heavy chain variable region (HCDR1, HCDR2, and HCDR3) and three in the light chain variable region (LCDR1, LCDR2, and LCDR3). A heavy-chain antibody or a single variable domain typically has three CDRs (CDR1, CDR2, and CDR3).

There are currently many ways to define CDRs. The Kabat scheme defines CDRs based on sequence variability and is the most commonly used (Elvin A. Kabat, et al., Sequences of Proteins of Immunological Interest, 5th Ed., Public Health Service, National Institutes of Health, Bethesda, Md. (1991)), while the Chothia scheme defines CDRs based on the position of structural loops (Cyrus Chothia, et al., Canonical Structures for the Hypervariable Regions of Immunoglobulins, J. Mol. Biol., 196:901-917(1987)). The AbM scheme, a compromise between the Kabat scheme and the Chothia scheme, is used by the Oxford Molecular's AbM antibody modeling software. "Contact" defines CDRs based on the analysis of the crystal structure of available complexes. However, it should be noted that boundaries of the CDRs of variable regions of the same antibody based on different methods and definitions may differ, that is, CDR sequences of the variable regions of the same antibody defined by different methods may differ. Thus, where reference is made to an antibody defined with a particular CDR sequence defined herein, the scope of the antibody also encompasses antibodies defined by CDR sequences that are converted to other arbitrary definitions (e.g., Chothia, AbM definitions, and the like).

The term "framework region" or "FR" refers to amino acid residues of variable domains other than the CDR residues defined herein.

The term "Fab" refers to a protein consisting of VH and CH1 domains of the heavy chain and VL and CL domains of the light chain of an immunoglobulin. The Fab herein refers to a Fab in its natural form or a modified Fab comprising a Fab heavy chain consisting of a heavy chain variable region and a constant region CH1 (VH-CH1, in a direction from N-terminus to C-terminus), and a Fab light chain consisting of a light chain variable region and a constant region CL (VL-CL, in a direction from N-terminus to C-terminus). The modified Fab may be, for example, a Fab introducing an amino acid substitution into the CH1/CL domain or/and the VH/VL domain. As a specific example, the modified Fab may be a Fab introducing an amino acid substitution into the CL domain.

The term "Fc domain" or "Fc" is used herein to define the C-terminal region of an immunoglobulin heavy chain, which comprises at least part of a constant region. The term includes natural sequence Fc and variant Fc. The C-terminal lysine (Lys447) of the Fc may or may not be present. Unless otherwise stated, amino acid residues in the Fc are numbered according to the EU numbering system, also referred to as the EU index, as described in Kabat, E.A. et al., Sequences of Proteins of Immunological Interest, 5th Ed., Public Health Service, National Institutes of Health, Bethesda, MD (1991), NIH Publication 91-3242. When the first paired domain and the second paired domain involve the description of amino acid positions, it is meant that they are numbered in sequence according to the starting amino acid of the corresponding sequence as the first position, for example, R60C substitution in SEQ ID NO: 1 means that R at position 60 in a sequence numbered starting from the first amino acid in SEQ ID NO: 1 as position 1 is substituted with C. As used herein, one of "Fc polypeptides" of an Fc domain refers to one of the two polypeptides that form a dimeric Fc domain. For example, the Fc polypeptide of an IgG Fc domain comprises IgG CH2 and IgG CH3 constant regions.

The term "KD" as used herein refers to the equilibrium dissociation constant, expressed in molar concentration (M). The KD value of an antibody can be determined using methods well known in the art. A method for determining the KD of an antibody is to use surface plasmon resonance, e.g., to use a biosensor system, such as a Biacore system. A method for determining the KD of an antibody is to use bio-layer interferometry (BLI), such as a ForteBio system.

The term "treating" or "treatment" refers to an attempt to alter the natural course of a disease in a treated individual, and may be a clinical intervention performed for prophylaxis or during the course of clinical pathology. Desired therapeutic effects include, but are not limited to, preventing the occurrence or recurrence of diseases, relieving symptoms, reducing any direct or indirect pathological outcomes of diseases, preventing metastasis, slowing disease progression rates, improving or alleviating disease conditions, and regressing or improving prognosis.

The term "subject" includes any human or non-human animal. The term "non-human animal" includes all vertebrates, e.g., mammals and non-mammals, such as non-human primates, sheep, dog, cat, horse, cow, chicken, amphibians, and reptiles. Preferably, the subject according to the present disclosure is a human. The terms "patient" and "subject" are used interchangeably unless otherwise indicated.

The term "isolated" means that a compound of interest (e.g., VHH, a multispecific antibody, an antibody, or a nucleic acid) has been isolated from its natural environment.

The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. Alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to obtain maximum alignment for the full length of sequences being compared.

In the context of the present disclosure, amino acid substitutions are represented as: original amino acid-position-substituted amino acid, using three-letter or single-letter codes, including codes Xaa and X to represent amino acid residues. Thus, for example, "H435R" means that amino acid H at position 435 is substituted with amino acid R, and more than 1 substituted amino acids may be contained, for example, T366Y/W means that amino acid T at position 366 is substituted with amino acid Y or W.

The term "include", "contain" or "comprise" and variants thereof should be understood as "including but not limited to", which means that other elements, components and steps that are not specified are encompassed in addition to the elements, components and steps that are listed.

Unless otherwise specified clearly herein, singular terms encompass plural terms, and vice versa.

All patents, patent applications and other identified publications are explicitly incorporated herein by reference for the purpose of description and . These publications are provided solely because they were disclosed prior to the filing date of the present application. All statements as to the dates of these documents or description as to the contents of these documents are based on the information available to the applicant and do not constitute any admission as to the correctness of the dates or the contents of these documents. Moreover, in any country or region, any reference to these publications herein is not to be construed as an admission that the publications form part of the commonly recognized knowledge in the art. Various aspects of the present disclosure will be described in further detail in the following sections.

Various aspects of the present disclosure will be described in further detail in the following sections.

### Bispecific antibody

According to the present disclosure, a constant region of one antigen-binding portion is engineered, and the constructed bispecific antibody can prevent or attenuate the mispairing of heavy and light chains with different specificities, thereby improving the yield or/and purity of the antibody of interest. Particularly, the α3 domain and β2 microglobulin of human MHC-I molecule are engineered, and the first paired domain and the second paired domain are constructed to replace the CH1 and CL domains of an antibody, so as to improve chain mispairing. For example, the first antigen-binding portion and the second antigen-binding portion are less likely to occur mispairing compared to the case where both the first and second antigen-binding portions are counterparts of a natural Fab.

The immunogenicity of the existing biological drugs is mainly divided into endogenous and exogenous. The endogenous immunogenicity is derived from non-human amino acid sequences, non-human structural forms, and a series of chemical modifications (point mutations, domain fusions, glycoform transformations, etc.). The exogenous immunogenicity is mainly derived from production process, drug delivery, genetic background of patients, dosage, frequency and mode of administration, and the like. With the increasing variety and quantity of biological drugs and the wide application thereof, problems associated with the immunogenicity are also gradually attracting high attention. Major histocompatibility complex (MHC) is the basis for recognition by the immune system and is involved in the adaptive immune process of the body. Bispecific antibodies designed based on the MHC molecule compositions have good compatibility with human body immune environment. Compared to MHC class II molecules, MHC class I molecules are more widely distributed, are expressed on the surface of almost all nucleated cells, and are recognized by CD8+ T cells. Among HLA-I molecules, the number of HLA-I-B class of human MHC-I molecules is the largest, and thus constant regions of the human MHC-I molecules based on the HLA-I-B class are selected and used for constructing structural elements of bispecific antibodies, which can reduce the risk of immunogenicity.

Furthermore, the constant regions of the human MHC-I molecules based on the HLA-I-B class do not contain N-linked and O-linked glycosylation sites, thereby having good developability and low immunogenicity.

It is found by experiments that the engineered bispecific antibodies still have good antigen-binding performance.

The present disclosure provides a bispecific antibody comprising a first antigen-binding portion and a second antigen-binding portion that specifically bind to two different antigens or different epitopes of the same antigen, wherein the first antigen-binding portion comprises:
a first polypeptide comprising, from N-terminus to C-terminus, a first heavy chain variable domain and a first paired domain operably linked to the first heavy chain variable domain, and
a second polypeptide comprising, from N-terminus to C-terminus, a first light chain variable domain and a second paired domain operably linked to the first light chain variable domain,
wherein one of the first paired domain and the second paired domain comprises an amino acid sequence of engineered HLA-I α3, and the other paired domain comprises an amino acid sequence of engineered β2 microglobulin.

In some embodiments, the first paired domain and the second paired domain can form a dimer, at least one non-natural interchain bond can be formed between the first paired domain and the second paired domain, and the non-natural interchain bond can stabilize the dimer.

The first paired domain and the second paired domain can form a dimer, which has good binding affinity to an antigen in different position orientations of the first antigen-binding portion (e.g., linked to a light chain variable region or a heavy chain variable region).

In one embodiment, the first paired domain comprises an amino acid sequence of the engineered HLA-I α3, and the second paired domain comprises an amino acid sequence of the engineered β2 microglobulin.

In another embodiment, the first paired domain comprises an amino acid sequence of the engineered β2 microglobulin, and the second paired domain comprises an amino acid sequence of the engineered HLA-I α3.

The non-natural interchain bond can stabilize the dimer formed by the first paired domain and the second paired domain, and amino acid residues forming the non-natural interchain bond are at the contact interface of the first paired domain and the second paired domain to effectively form the bond. The term "contact interface" refers to a particular region on the polypeptides where the polypeptides interact/associate with each other. A contact interface comprises one or more amino acid residues that are capable of interacting with the corresponding amino acid residues in contact or association when interaction occurs. The amino acid residues in a contact interface may or may not be in a continuous sequence. For example, when the interface is three-dimensional, the amino acid residues within the interface can be separated at different positions on the linear sequence.

The number of the non-natural interchain bonds is 1-3, for example, may be 1, 2, or 3, and preferably, the number of the non-natural interchain bonds is 1 in order to resemble a natural antibody as much as possible. In one embodiment, the non-natural interchain bond is a disulfide bond.

In some embodiments, the amino acid sequence of the engineered HLA-I α3 has at least 85%, 86%, 87%, 88%, 89%, 90%, 91%, 92%, 93%, 94%, 95%, 96%, 97%, 98%, 99%, or 100% identity to a sequence set forth in SEQ ID NO: 1, and the amino acid sequence of the engineered β2 microglobulin has at least 85%, 86%, 87%, 88%, 89%, 90%, 91%, 92%, 93%, 94%, 95%, 96%, 97%, 98%, 99%, or 100% identity to a sequence set forth in SEQ ID NO: 2. In some embodiments, the amino acid sequences of the engineered HLA-I α3 and the engineered β2 microglobulin have at least 95%, 96%, 97%, 98%, 99%, or 100% identity to sequences set forth in SEQ ID NO: 1 and SEQ ID NO: 2, respectively.

The sequence set forth in SEQ ID NO: 1 is derived from a consensus sequence generated by aligning constant region sequences of a plurality of HLA-I-B class molecules, and the paired domains formed by utilizing or modifying the consensus sequence have good universality.

In some embodiments, the engineered HLA-I α3 comprises an amino acid sequence having an amino acid substitution in the sequence set forth in SEQ ID NO: 1, and the engineered β2 microglobulin comprises an amino acid sequence having an amino acid substitution in the sequence set forth in SEQ ID NO: 2.

The amino acid substitutions in the engineered HLA-I α3 and the engineered β2 microglobulin comprise amino acid substitutions that form a non-natural interchain bond (e.g., a disulfide bond) or/and amino acid substitutions that improve the dimer formed by the paired domains or the bispecific antibody.

In some embodiments, the amino acid substitutions in both the engineered HLA-I α3 and the engineered β2 microglobulin comprise cysteine residue substitutions that occur at a contact interface between the two and can form a disulfide bond with each other. In a specific embodiment, the cysteine residue substitution is R60C in SEQ ID NO: 1 and Y26C in SEQ ID NO: 2. In a specific embodiment, the cysteine residue substitution is A62C in SEQ ID NO: 1 and R12C in SEQ ID NO: 2. In a specific embodiment, the cysteine residue substitution is G63C in SEQ ID NO: 1 and Y67C in SEQ ID NO: 2. In other specific embodiments, the cysteine residue substitutions may be selected from two or three pairs of R60C/Y26C, A62C/R12C, and G63C/Y67C described above.

In some embodiments, the amino acid substitutions in the engineered HLA-I α3 or/and the engineered β2 microglobulin comprise amino acid substitutions that increase an isoelectric point of the dimer formed by the paired domains or the bispecific antibody. These amino acid substitutions may occur simultaneously in both paired domains, or in either of the paired domains. Generally, the isoelectric point of the dimer formed by the paired domains or the bispecific antibody is increased to 6.5-9.0, so as to facilitate the development of the production process and improve the druggability. In some embodiments, the isoelectric point of the dimer formed by the paired domains or the bispecific antibody is increased to 6.5-8.5, 7.0-8.5, 7.0-9.0, 7.0-7.8, 7.0-8.0, 7.5-7.8, or 7.5-8.0. In some specific embodiments, the isoelectric point of the dimer formed by the paired domains or the bispecific antibody is increased to 6.5, 6.7, 6.9, 7.1, 7.3, 7.5, 7.7, 7.8, 7.9, 8.0, 8.2, 8.3, 8.5, 8.7, or 9.0. The isoelectric point can be determined experimentally or calculated theoretically. The theoretical isoelectric point can be calculated, for example, using an online computational analysis tool Expasy-Compute pI/Mw tool (http://web.expasy.org/compute_pi/).

In some embodiments, the amino acid substitutions that increase the isoelectric point comprise a substitution in the engineered HLA-I α3 with a positively charged amino acid at one or more positions of E3, D22, E48, D53, E90, and E101 in the sequence set forth in SEQ ID NO: 1.

In some embodiments, the amino acid substitutions that increase the isoelectric point comprise a substitution in the engineered β2 microglobulin with a positively charged amino acid at one or more positions of E74, E47, E69, D34, E16, D53, E44, E50, and E36 in the sequence set forth in SEQ ID NO: 2.

In some specific embodiments, the amino acid substitutions that increase the isoelectric point comprise: a substitution in the engineered HLA-I α3 with a positively charged amino acid at positions D22, E48, and D53 in the sequence set forth in SEQ ID NO: 1, and a substitution in the engineered β2 microglobulin with a positively charged amino acid at position E69 in the sequence set forth in SEQ ID NO: 2.

In some embodiments, the positively charged amino acid is K or R.

In a more specific embodiment, the amino acid substitutions that increase the isoelectric point comprise: amino acid substitutions D22R, E48K, and D53K comprised in the sequence set forth in SEQ ID NO: 1 for the engineered HLA-I α3, and an amino acid substitution E69R comprised in the sequence set forth in SEQ ID NO: 2 for the engineered β2 microglobulin.

In some more specific embodiments, the amino acid substitutions comprise: amino acid substitutions A62C, D22R, E48K, and D53K comprised in the sequence set forth in SEQ ID NO: 1 for the engineered HLA-I α3, and amino acid substitutions R12C and E69R comprised in the sequence set forth in SEQ ID NO: 2 for the engineered β2 microglobulin. In a further embodiment, the engineered β2 microglobulin contains "GP" added at the carboxyl-terminus in the sequence set forth in SEQ ID NO: 2.

In a specific embodiment, the engineered HLA-I α3 comprises an amino acid sequence set forth in SEQ ID NO: 3, and the engineered β2 microglobulin comprises an amino acid sequence set forth in SEQ ID NO: 4.

In another specific embodiment, the engineered HLA-I α3 comprises an amino acid sequence set forth in SEQ ID NO: 3, and the engineered β2 microglobulin comprises an amino acid sequence set forth in SEQ ID NO: 5.

In some embodiments, the second antigen-binding portion comprises a second heavy chain variable domain and a second light chain variable domain. The second antigen-binding portion does not comprise the first paired domain and the second paired domain described herein. In a specific embodiment, the second antigen-binding portion comprises a Fab. The first antigen-binding portion and the second antigen-binding portion specifically bind to two different antigens or different epitopes of the same antigen. In some embodiments, both the first antigen-binding portion and the second antigen-binding portion are monovalent binding to the respective antigens.

In some embodiments, the CL domain of the Fab is substituted with a hydrophobic amino acid at one or more of positions T/S114, T/F116, and F118 according to the EU numbering. In some embodiments, the hydrophobic amino acid is selected from a non-aromatic group such as methionine, alanine, valine, isoleucine, or leucine. The mispairing of the light and heavy chains can be further reduced by introducing suitable amino acid substitutions in the CL domain of the Fab of the second antigen-binding portion. In a specific embodiment, an amino acid in the CL domain of the Fab is substituted with alanine (A) at position F118 according to the EU numbering. In other embodiments, an amino acid in the CL domain of the Fab is substituted with alanine at position F118 according to the EU numbering, and the CL domain further comprises other substitutions, for example, substitutions that further reduce the mispairing of the CL with a paired domain comprising the engineered HLA-I α3.

In some embodiments, the CL domain may be derived from a human κ light chain CL domain or a human λ light chain CL domain. In a specific embodiment, the CL domain comprising F118A comprises an amino acid sequence set forth in SEQ ID NO: 44. In a specific embodiment, the amino acid sequence of the CL domain comprising F118A is set forth in SEQ ID NO: 44.

In some embodiments, the CH1 domain of the Fab may be a CH1 domain of human IgG (IgG1-4), IgM, IgA, IgD, or IgE. In a specific embodiment, the CH1 domain of the Fab is a CH1 domain of human IgG1. In a specific embodiment, the CH1 domain comprises an amino acid sequence set forth in SEQ ID NO: 34. In a specific embodiment, the amino acid sequence of the CH1 domain is set forth in SEQ ID NO: 34. In a specific embodiment, the CH1 domain of the Fab is a CH1 domain of human IgG4.

The amino acid sequence set forth in SEQ ID NO: 34 is as follows:

ASTKGPSVFPLAPSSKSTSGGTAALGCLVKDYFPEPVTVSWNSGALTSGVHTFPAVLQSSG
LYSLSSVVTVPSSSLGTQTYICNVNHKPSNTKVDKKV

The amino acid sequence set forth in SEQ ID NO: 35 is as follows:

The amino acid sequence set forth in SEQ ID NO: 44 is as follows:

RTVAAPSVFIAPPSDEQLKSGTASVVCLLNNFYPREAKVQWKVDNALQSGNSQESVTEQD
SKDSTYSLSSTLTLSKADYEKHKVYACEVTHQGLSSPVTKSFNRGEC

In some specific embodiments, an amino acid in the CL domain of the Fab is substituted with alanine at position F118, and the CH1 domain of the Fab does not comprise an amino acid substitution at position 128 (EU numbering), position 141 (EU numbering), position 185 (EU numbering), position 11 (IMGT numbering), position 28 (IMGT numbering), or position 139 (Kabat numbering). In other embodiments, an amino acid in the CL domain of the Fab is substituted with alanine at position F118 according to the EU numbering, and the CL domain further comprises other substitutions (for example, substitutions that further reduce the mispairing of the CL with a paired domain comprising the engineered HLA-I α3). The CH1 domain of the Fab does not comprise an amino acid substitution at position 128 (EU numbering), position 141 (EU numbering), position 185 (EU numbering), position 11 (IMGT numbering), position 28 (IMGT numbering), or position 139 (Kabat numbering).

In some specific embodiments, an amino acid in the CL domain of the Fab is substituted with alanine at position F118, and the CH1 domain of the Fab does not comprise an amino acid substitution L128F (EU numbering), A141L (EU numbering), V185A/L (EU numbering), L11K/F/W (IMGT numbering), L28N/R (IMGT numbering), or A139W/V/I (Kabat numbering). In other embodiments, an amino acid in the CL domain of the Fab is substituted with alanine at position F118 according to the EU numbering, and the CL domain further comprises other substitutions (for example, substitutions that further reduce the mispairing of the CL with a paired domain comprising the engineered HLA-I α3). The CH1 domain of the Fab does not comprise an amino acid substitution L128F (EU numbering), A141L (EU numbering), V185A/L (EU numbering), L11K/F/W (IMGT numbering), L28N/R (IMGT numbering), or A139W/V/I (Kabat numbering).

In some specific embodiments, an amino acid in the CL domain of the Fab is substituted with alanine at position F118, and the CH1 domain of the Fab uses a CH1 domain of a wild-type immunoglobulin, e.g., a CH1 domain of human IgG. In other embodiments, an amino acid in the CL domain of the Fab is substituted with alanine at position F118 according to the EU numbering, and the CL domain further comprises other substitutions (for example, substitutions that further reduce the mispairing of the CL with a paired domain comprising the engineered HLA-I α3). The CH1 domain of the Fab uses a CH1 domain of a wild-type immunoglobulin, e.g., a CH1 domain of human IgG (IgG1, IgG2, IgG3, or IgG4).

In some specific embodiments, the CL domain of the Fab comprises an amino acid sequence having at least 85%, 86%, 87%, 88%, 89%, 90%, 91%, 92%, 93%, 94%, 95%, 96%, 97%, 98%, 99%, or 100% identity to the sequence set forth in SEQ ID NO: 44, and the CH1 domain of the Fab comprises an amino acid sequence having at least 85%, 86%, 87%, 88%, 89%, 90%, 91%, 92%, 93%, 94%, 95%, 96%, 97%, 98%, 99%, or 100% identity to the sequence set forth in SEQ ID NO: 34. Further, in this embodiment, the CL domain of the Fab comprises F118A. Further, in this embodiment, the CH1 domain of the Fab is a CH1 domain of a wild-type immunoglobulin, or the CH1 domain of the Fab does not comprise an amino acid substitution at position 128 (EU numbering), position 141 (EU numbering), position 185 (EU numbering), position 11 (IMGT numbering), position 28 (IMGT numbering), or position 139 (Kabat numbering).

In some specific embodiments, the CL domain of the Fab comprises an amino acid sequence set forth in SEQ ID NO: 44, and the CH1 domain of the Fab comprises an amino acid sequence set forth in SEQ ID NO: 34.

In some specific embodiments, the amino acid sequence of the CL domain of the Fab is set forth in SEQ ID NO: 44, and the amino acid sequence of the CH1 domain of the Fab is set forth in SEQ ID NO: 34.

In some embodiments, one of the first antigen-binding portion and the second antigen-binding portion binds to a T cell-specific receptor molecule and/or a natural killer cell-specific receptor molecule, and the other antigen-binding portion binds to a tumor-associated antigen.

The T cell-specific receptor molecule allows T cells to bind and, if additional signals are present, is activated by and responds to an epitope/antigen presented by another cell referred to as an antigen-presenting cell or APC. The T cell-specific receptor molecule may be TCR, CD3, CD28, CD134 (also referred to as OX40), 4-1BB, CD5, or CD95 (also referred to as Fas receptor).

Examples of the NK cell-specific receptor molecule include CD16, a low-affinity Fc receptor and NKG2D, and CD2.

The term "tumor-associated antigen" refers to an antigen that is presented on the surface of a tumor cell or may be presented on the surface of a tumor cell and located on or within the tumor cell. In some embodiments, the tumor-associated antigen may be presented only by tumor cells, rather than by normal, i.e., non-tumor, cells. In some other embodiments, the tumor-associated antigen may be expressed only on tumor cells, or may represent a tumor-specific mutation as compared to non-tumor cells. In some other embodiments, the tumor-associated antigen may be found in both tumor cells and non-tumor cells, but is overexpressed on the tumor cells as compared to the non-tumor cells, or may bind to an antibody in the tumor cells due to the less compact structure of tumor tissues as compared to non-tumor tissues. In some embodiments, the tumor-associated antigen is located on the vasculature of a tumor. Some exemplary tumor-associated antigens are CD38, CD47, PD-L1, PD-1, and the like.

In some embodiments, one of the first antigen-binding portion and the second antigen-binding portion specifically binds to CD3, and the other specifically binds to a tumor-associated antigen. For example, the first antigen-binding portion specifically binds to CD3, and the second antigen-binding portion specifically binds to a tumor-associated antigen; or the second antigen-binding portion specifically binds to CD3, and the first antigen-binding portion specifically binds to a tumor-associated antigen. In some specific embodiments, the first antigen-binding portion specifically binds to CD3, and the second antigen-binding portion specifically binds to CD38; or the first antigen-binding portion specifically binds to CD38, and the second antigen-binding portion specifically binds to CD3.

In some embodiments, both the first antigen-binding portion and the second antigen-binding portion specifically bind to tumor-associated antigens. In some specific embodiments, the first antigen-binding portion specifically binds to PD-L1, and the second antigen-binding portion specifically binds to CD47; or the first antigen-binding portion specifically binds to CD47, and the second antigen-binding portion specifically binds to PD-L1.

In some embodiments, the bispecific antibody further comprises an Fc domain composed of two Fc polypeptides capable of stable association. In some embodiments, the bispecific antibody is bivalent.

In some embodiments, the first antigen-binding portion is operably linked at its C-terminus to N-terminus of one of the Fc polypeptides, and the second antigen-binding portion is operably linked at its C-terminus to N-terminus of the other Fc polypeptide. In some more specific embodiments, the first polypeptide of the first antigen-binding portion is operably linked at its C-terminus to N-terminus of one of the Fc polypeptides, and the second antigen-binding portion comprises a Fab and a Fab heavy chain of the second antigen-binding portion is operably linked at its C-terminus to N-terminus of the other Fc polypeptide. In some more specific embodiments, the first polypeptide of the first antigen-binding portion is operably linked at its C-terminus to N-terminus of one of the Fc polypeptides, and the second antigen-binding portion comprises a Fab and a Fab light chain of the second antigen-binding portion is operably linked at its C-terminus to N-terminus of the other Fc polypeptide.

In some embodiments, the bispecific antibody further comprises a third antigen-binding portion. In some specific embodiments, the binding of the bispecific antibody to an antigen is trivalent.

In some embodiments, the third antigen-binding portion binds to the same antigen epitope as the second antigen-binding portion; preferably, the third antigen-binding portion is identical to the second antigen-binding portion. In other embodiments, the third antigen-binding portion binds to the same antigen epitope as the first antigen-binding portion; preferably, the third antigen-binding portion is identical to the first antigen-binding portion.

In some embodiments, the third antigen-binding portion is operably linked to N-terminus or C-terminus of the first antigen-binding portion.

In other embodiments, the third antigen-binding portion is operably linked to N-terminus or C-terminus of the second antigen-binding portion.

In some embodiments, the bispecific antibody comprises a third antigen-binding portion and further comprises an Fc domain composed of two Fc polypeptides capable of stable association.

In some specific embodiments, the first antigen-binding portion is operably linked at its C-terminus to N-terminus of one of the Fc polypeptides, the second antigen-binding portion is operably linked at its C-terminus to N-terminus of the other Fc polypeptide, and the third antigen-binding portion is operably linked at its C-terminus to N-terminus of the first antigen-binding portion or N-terminus of the second antigen-binding portion.

In other specific embodiments, the third antigen-binding portion is operably linked at its C-terminus to N-terminus of one of the Fc polypeptides, the first antigen-binding portion is operably linked at its C-terminus to N-terminus of the third antigen-binding portion, and the second antigen-binding portion is operably linked at its C-terminus to N-terminus of the other Fc polypeptide.

In other specific embodiments, the third antigen-binding portion is operably linked at its C-terminus to N-terminus of one of the Fc polypeptides, the second antigen-binding portion is operably linked at its C-terminus to N-terminus of the third antigen-binding portion, and the first antigen-binding portion is operably linked at its C-terminus to N-terminus of the other Fc polypeptide.

The third antigen-binding portion described above may be operably linked to the first antigen-binding portion by a peptide linker.

The third antigen-binding portion described above may be operably linked to the second antigen-binding portion by a peptide linker. The peptide linker may be any suitable, e.g., electrically charged and/or flexible, linker polypeptide. In a specific embodiment, the peptide linker consists of 1 to 50 amino acids linked by peptide bonds, wherein the amino acids may be selected from 20 naturally occurring amino acids; in a more preferred embodiment, the 1 to 50 amino acids are selected from glycine, alanine, proline, serine, asparagine, glutamine, and lysine. Thus, exemplary peptide linkers may be polyglycines (particularly (Gly)4 and (Gly)5), poly(Gly-Ser), (Gly)3AsnGlySer(Gly)2, (Gly)3Cys(Gly)4, GlyProAsnGlyGly, or those disclosed in Table 4 of patent application WO2019195535, etc. In some embodiments, the peptide linker may be a peptide linker consisting of glycine and serine. In some embodiments, the peptide linker may comprise 0, 1, 2, 3, 4, 5, 6, 7, 8, 9, 10, 11, 12, 13, 14, 15, 16, 17, 18, 19, 20 or more amino acids.

When the antigen-binding portion is operably linked to the Fc domain or Fc polypeptide, it is typically linked by a hinge region. The hinge, for example, comprises amino acids of the hinge region of human IgG.

### Fc domain

An Fc domain of a bispecific antibody consists of a pair of polypeptide chains comprising a heavy chain domain of an immunoglobulin molecule. For example, the Fc domain of immunoglobulin G (IgG) molecule is a dimer, and each of Fc polypeptides comprises CH2 and CH3 IgG heavy chain constant regions. The two Fc polypeptides of the Fc domain are capable of stable association with each other. For example, the two Fc polypeptides are stably associated by one or more of a linker, a disulfide bond, a hydrogen bond, an electrostatic interaction, a salt bridge, and a hydrophobic-hydrophilic interaction. In one embodiment, the bispecific antibody of the present disclosure comprises one Fc domain.

In one embodiment, the Fc domain of the bispecific antibody is an IgG Fc domain. In a specific embodiment, the Fc domain is an IgG1 Fc domain. In another embodiment, the Fc domain is an IgG4 Fc domain. In a more specific embodiment, the Fc domain is an IgG4 Fc domain comprising an amino acid substitution at position S228, particularly amino acid substitution S228P, which reduces the *in vivo* Fab arm exchange for an IgG4 antibody. In yet another specific embodiment, the Fc domain is a human Fc domain. In a more specific embodiment, the Fc domain is a human IgG1 Fc domain.

In some embodiments, the Fc domain comprises a modification, such as an amino acid substitution. The modification may be, for example, a modification that promotes heterodimerization, a modification that alters effector function, a modification that alters the binding ability to protein A, or the like.

In some embodiments, the Fc domain comprises a modification that promotes heterodimerization. The bispecific antibody of the present disclosure comprises different antigen-binding portions fused to one or the other of the two Fc polypeptides in the Fc domain, thus, the two Fc polypeptides are typically comprised in two different polypeptide chains. Several possible combinations of the two polypeptides are generated by recombinant co-expression and subsequent dimerization of these polypeptides. In order to increase the yield and purity of the multispecific antibody in recombinant production, it would be advantageous to introduce a modification that promotes the binding of the desired polypeptides into the Fc domain of the bispecific antibody. Thus, in a specific embodiment, the Fc domain comprises an amino acid substitution that promotes association of the two Fc polypeptides of the Fc domain.

The site for the most extensive protein-protein interaction between the two Fc polypeptides of the human IgG Fc domain is in the CH3 domain of the Fc domain. Thus, in one embodiment, the modification is in the CH3 domain of the Fc domain.

In a specific embodiment, the modification is a so-called "knob-into-hole" modification, which comprises a "knob" modification in one of the two Fc polypeptides of the Fc domain and a "hole" modification in the other one of the two Fc polypeptides of the Fc domain. Generally, the method involves introducing a protuberance ("knob") at the interface of one Fc polypeptide and a corresponding cavity ("hole") at the interface of the other Fc polypeptide, such that the protuberance can be positioned in the cavity to promote heterodimer formation and to interfere with homodimer formation. The protuberance is constructed by substituting a small amino acid side chain from the interface of one Fc polypeptide with a larger side chain (e.g., tyrosine or tryptophan, etc.). The complementary cavity having the same or similar size as the protuberance is created in the interface of the other Fc polypeptide by substituting a large amino acid side chain with a smaller amino acid side chain (e.g., alanine or threonine, etc.).

Thus, in a specific embodiment, an amino acid residue is substituted with an amino acid residue having a larger side chain volume in the CH3 domain of one Fc polypeptide of the bispecific antibody, thereby creating a protuberance within the CH3 domain of the Fc polypeptide that can be positioned in a cavity within the CH3 domain of the other Fc polypeptide, and an amino acid residue is substituted with an amino acid residue having a smaller side chain volume in the CH3 domain of the other Fc polypeptide, thereby creating a cavity within the CH3 domain of the Fc polypeptide.

In some specific embodiments, one Fc polypeptide of the Fc domain comprises T366Y/W or/and S354C, and the other Fc polypeptide comprises Y407T/V, Y349C, T366S, or/and L368A. In a more specific embodiment, one of the Fc polypeptides of the Fc domain comprises amino acid substitutions T366Y/W and S354C, and the other Fc polypeptide comprises amino acid substitutions Y407T/V, Y349C, T366S, and L368A. In a more specific embodiment, the Fc may be an Fc of human IgG1.

In some embodiments, the Fc domain comprises a modification that reduces or eliminates the binding of the CH3 region of one Fc polypeptide in the Fc domain to Protein A (from *Staphylococcus aureus*). In some embodiments, the modification is an amino acid substitution. In some embodiments, the Fc domain comprises an amino acid substitution H435R or/and Y436F, which occurs only in one Fc polypeptide rather than in the other Fc polypeptide. In a specific embodiment, the Fc domain comprises an amino acid substitution H435R or/and Y436F, which occurs only in one of the Fc polypeptides. In one such specific embodiment, the Fc is IgG1 Fc, particularly human IgG1 Fc.

### Composition

The present disclosure provides a pharmaceutical composition comprising the bispecific antibody and further comprising one or more pharmaceutically acceptable carriers. The pharmaceutically acceptable carriers include, for example, excipients, diluents, encapsulating materials, fillers, buffers, or other agents.

### Isolated Nucleic Acid

The present disclosure provides an isolated polynucleotide encoding the bispecific antibody. The nucleic acid sequences of the polypeptide chains of some bispecific antibodies are illustratively listed in the sequence listing.

### Vector

The present disclosure provides an isolated vector comprising the polynucleotide. In some embodiments, the vector is a cloning vector; in other embodiments, the vector is an expression vector; in a specific embodiment, the expression vector is pcDNA3.1. The expression vector is optionally any expression vector capable of expressing the multispecific antibody described herein.

### Host cell

In some embodiments, the present disclosure provides a host cell comprising the vector or the polynucleotide described herein, the host cell being a suitable host cell for use in cloning or encoding a multispecific antibody. In some embodiments, the host cell is a prokaryotic cell. In other embodiments, the host cell is a eukaryotic cell. In some embodiments, the host cell is selected from a yeast cell, a mammalian cell, or other cells suitable for preparing a multispecific antibody. The mammalian cell is, for example, Chinese hamster ovary (CHO) cells or CHO-S cells.

### Methods for expressing bispecific antibody

The present disclosure provides a method for expressing the bispecific antibody described herein comprising culturing the host cell under such conditions that the bispecific antibody is expressed. To produce the bispecific antibody, a polynucleotide encoding the bispecific antibody is isolated and inserted into one or more vectors for further use in cloning or/and expression in a host cell. The polynucleotide can be obtained using various methods known in the art, such as gene splicing and chemical synthesis.

### Conjugate

The present disclosure provides a conjugate comprising the bispecific antibody described herein and a therapeutic agent linked or conjugated to the bispecific antibody.

### Fusion protein

The present disclosure provides a fusion protein, wherein a fusion component is expressed by fusion with the bispecific antibody described herein. The construction and expression of the fusion protein molecule can be achieved by genetic engineering means.

### Use

The present disclosure provides a method of treating a disease in a subject in need thereof, comprising administering to the subject a therapeutically effective amount of the bispecific antibody, the pharmaceutical composition, the conjugate, or the fusion protein described herein.

The disease may be cancer, and non-limiting examples of some cancers are selected from leukemia, lymphoma, myeloma, ovarian cancer, breast cancer, endometrial cancer, colon cancer, rectal cancer, kidney cancer, bladder cancer, urothelial cancer, lung cancer, bronchial cancer, bone cancer, prostate cancer, pancreatic cancer, gastric cancer, hepatocellular carcinoma, gallbladder cancer, bile duct cancer, esophageal cancer, renal cell cancer, thyroid cancer, head and neck cancer, testicular cancer, endocrine adenocarcinoma, adrenal cancer, pituitary gland cancer, skin cancer, soft tissue cancer, vascular cancer, brain cancer, nerve cancer, eye cancer, meningeal cancer, oropharyngeal cancer, hypopharynx cancer, cervical cancer, uterine cancer, glioblastoma, medulloblastoma, astrocytoma, glioma, meningioma, gastrinoma, neuroblastoma, melanoma, myelodysplastic syndrome, and sarcoma.

The present disclosure further provides the following embodiments, but is not limited thereto:
Embodiment 1. A bispecific antibody, comprising a first antigen-binding portion and a second antigen-binding portion that specifically bind to two different antigens or different epitopes of the same antigen, wherein the first antigen-binding portion comprises:
   a first polypeptide comprising, from N-terminus to C-terminus, a first heavy chain variable domain and a first paired domain operably linked to the first heavy chain variable domain, and
   a second polypeptide comprising, from N-terminus to C-terminus, a first light chain variable domain and a second paired domain operably linked to the first light chain variable domain, wherein one of the first paired domain and the second paired domain comprises an amino acid sequence of engineered HLA-I α3, and the other paired domain comprises an amino acid sequence of engineered β2 microglobulin.
Embodiment 2. The bispecific antibody according to Embodiment 1, wherein the first paired domain and the second paired domain can form a dimer, at least one non-natural interchain bond can be formed between the first paired domain and the second paired domain, and the non-natural interchain bond can stabilize the dimer.
Embodiment 3. The bispecific antibody according to Embodiment 1 or 2, wherein the first paired domain comprises an amino acid sequence of the engineered HLA-I α3, and the second paired domain comprises an amino acid sequence of the engineered β2 microglobulin.
Embodiment 4. The bispecific antibody according to Embodiment 1 or 2, wherein the first paired domain comprises an amino acid sequence of the engineered β2 microglobulin, and the second paired domain comprises an amino acid sequence of the engineered HLA-I α3.
Embodiment 5. The bispecific antibody according to any one of Embodiments 1 to 4, wherein the number of non-natural interchain bond is 1-3, preferably 1.
Embodiment 6. The bispecific antibody according to any one of Embodiments 1 to 5, wherein amino acid residues forming the non-natural interchain bond are at a contact interface of the first paired domain and the second paired domain.
Embodiment 7. The bispecific antibody according to any one of Embodiments 1 to 6, wherein the non-natural interchain bond is a disulfide bond.
Embodiment 8. The bispecific antibody according to any one of Embodiments 1 to 7, wherein the amino acid sequence of the engineered HLA-I α3 has at least 85%, 86%, 87%, 88%, 89%, 90%, 91%, 92%, 93%, 94%, 95%, 96%, 97%, 98%, 99%, or 100% identity to a sequence set forth in SEQ ID NO: 1, and the amino acid sequence of the engineered β2 microglobulin has at least 85%, 86%, 87%, 88%, 89%, 90%, 91%, 92%, 93%, 94%, 95%, 96%, 97%, 98%, 99%, or 100% identity to a sequence set forth in SEQ ID NO: 2.
Embodiment 9. The bispecific antibody according to any one of Embodiments 1 to 8, wherein the engineered HLA-I α3 comprises an amino acid sequence having an amino acid substitution in the sequence set forth in SEQ ID NO: 1, and the engineered β2 microglobulin comprises an amino acid sequence having an amino acid substitution in the sequence set forth in SEQ ID NO: 2.
Embodiment 10. The bispecific antibody according to Embodiment 9, wherein the amino acid substitutions in both the engineered HLA-I α3 and the engineered β2 microglobulin comprise cysteine residue substitutions that occur at a contact interface between the two and can form a disulfide bond with each other.
Embodiment 11. The bispecific antibody according to Embodiment 10, wherein the cysteine residue substitution is selected from one or more pairs in the following group:
   (1) R60C in SEQ ID NO: 1 and Y26C in SEQ ID NO: 2;
   (2) A62C in SEQ ID NO: 1 and R12C in SEQ ID NO: 2; and
   (3) G63C in SEQ ID NO: 1 and Y67C in SEQ ID NO: 2.
Embodiment 12. The bispecific antibody according to any one of Embodiments 9 to 11, wherein the amino acid substitutions in the engineered HLA-I α3 or/and the engineered β2 microglobulin comprise amino acid substitutions that increase an isoelectric point of the dimer formed by the paired domains or the bispecific antibody.
Embodiment 13. The bispecific antibody according to Embodiment 12, wherein the isoelectric point of the dimer formed by the paired domains or the bispecific antibody is increased to 6.5-9.0.
Embodiment 14. The bispecific antibody according to any one of Embodiments 12 to 13, wherein the amino acid substitutions that increase the isoelectric point comprise a substitution in the engineered HLA-I α3 with a positively charged amino acid at one or more positions of E3, D22, E48, D53, E90, and E101 in the sequence set forth in SEQ ID NO: 1.
Embodiment 15. The bispecific antibody according to any one of Embodiments 12 to 14, wherein the amino acid substitutions that increase the isoelectric point comprise a substitution in the engineered β2 microglobulin with a positively charged amino acid at one or more positions of E74, E47, E69, D34, E16, D53, E44, E50, and E36 in the sequence set forth in SEQ ID NO: 2.
Embodiment 16. The bispecific antibody according to Embodiment 15, wherein the amino acid substitutions that increase the isoelectric point comprise: a substitution in the engineered HLA-I α3 with a positively charged amino acid at positions D22, E48, and D53 in the sequence set forth in SEQ ID NO: 1, and a substitution in the engineered β2 microglobulin with a positively charged amino acid at position E69 in the sequence set forth in SEQ ID NO: 2.
Embodiment 17. The bispecific antibody according to any one of Embodiments 14 to 16, wherein the positively charged amino acid is K or R.
Embodiment 18. The bispecific antibody according to any one of Embodiments 12 to 13, wherein the amino acid substitutions that increase the isoelectric point comprise: amino acid substitutions D22R, E48K, and D53K comprised in the sequence set forth in SEQ ID NO: 1 for the engineered HLA-I α3, and an amino acid substitution E69R comprised in the sequence set forth in SEQ ID NO: 2 for the engineered β2 microglobulin.
Embodiment 19. The bispecific antibody according to any one of Embodiments 1 to 18, wherein the engineered HLA-I α3 comprises an amino acid sequence set forth in SEQ ID NO: 3, and the engineered β2 microglobulin comprises an amino acid sequence set forth in SEQ ID NO: 4.
Embodiment 20. The bispecific antibody according to any one of Embodiments 1 to 18, wherein the engineered HLA-I α3 comprises an amino acid sequence set forth in SEQ ID NO: 3, and the engineered β2 microglobulin comprises an amino acid sequence set forth in SEQ ID NO: 5.
Embodiment 21. The bispecific antibody according to any one of Embodiments 1 to 20, wherein the second antigen-binding portion comprises a second heavy chain variable domain and a second light chain variable domain.
Embodiment 22. The bispecific antibody according to any one of Embodiments 1 to 21, wherein the second antigen-binding portion comprises a Fab.
Embodiment 23. The bispecific antibody according to Embodiment 22, wherein an amino acid in a CL domain of the Fab is substituted with alanine at position F118 according to an EU numbering.
Embodiment 24. The bispecific antibody according to Embodiment 23, the CL domain comprises an amino acid sequence set forth in SEQ ID NO: 44.
Embodiment 25. The bispecific antibody according to any one of Embodiments 1 to 24, wherein one of the first antigen-binding portion and the second antigen-binding portion binds to a T cell-specific receptor molecule and/or a natural killer cell-specific receptor molecule, and the other antigen-binding portion binds to a tumor-associated antigen.
Embodiment 26. The bispecific antibody according to any one of Embodiments 1 to 24, wherein one of the first antigen-binding portion and the second antigen-binding portion specifically binds to CD3, and the other specifically binds to a tumor-associated antigen.
Embodiment 27. The bispecific antibody according to any one of Embodiments 1 to 24, wherein both the first antigen-binding portion and the second antigen-binding portion specifically bind to tumor-associated antigens.
Embodiment 28. The bispecific antibody according to any one of Embodiments 25 to 27, wherein the tumor-associated antigen is CD38, CD47, PD-L1, or PD-1.
Embodiment 29. The bispecific antibody according to Embodiment 26, wherein the first antigen-binding portion specifically binds to CD3, and the second antigen-binding portion specifically binds to CD38; or the first antigen-binding portion specifically binds to CD38, and the second antigen-binding portion specifically binds to CD3.
Embodiment 30. The bispecific antibody according to Embodiment 27, wherein the first antigen-binding portion specifically binds to PD-L1, and the second antigen-binding portion specifically binds to CD47; or the first antigen-binding portion specifically binds to CD47, and the second antigen-binding portion specifically binds to PD-L1.
Embodiment 31. The bispecific antibody according to any one of Embodiments 1 to 30, wherein the bispecific antibody is bivalent.
Embodiment 32. The bispecific antibody according to any one of Embodiments 1 to 30, further comprising a third antigen-binding portion, wherein the third antigen-binding portion binds to the same antigen epitope as the second antigen-binding portion; preferably, the third antigen-binding portion is identical to the second antigen-binding portion.
Embodiment 33. The bispecific antibody according to any one of Embodiments 1 to 30, further comprising a third antigen-binding portion, wherein the third antigen-binding portion binds to the same antigen epitope as the first antigen-binding portion; preferably, the third antigen-binding portion is identical to the first antigen-binding portion.
Embodiment 34. The bispecific antibody according to Embodiment 32 or 33, wherein the third antigen-binding portion is operably linked to N-terminus or C-terminus of the first antigen-binding portion.
Embodiment 35. The bispecific antibody according to Embodiment 32 or 33, wherein the third antigen-binding portion is operably linked to N-terminus or C-terminus of the second antigen-binding portion.
Embodiment 36. The bispecific antibody according to any one of Embodiments 32 to 35, wherein the bispecific antibody is trivalent.
Embodiment 37. The bispecific antibody according to any one of Embodiments 1 to 31, further comprising an Fc domain composed of two Fc polypeptides capable of stable association.
Embodiment 38. The bispecific antibody according to Embodiment 37, wherein the first antigen-binding portion is operably linked at its C-terminus to N-terminus of one of the Fc polypeptides, and the second antigen-binding portion is operably linked at its C-terminus to N-terminus of the other Fc polypeptide.
Embodiment 39. The bispecific antibody according to Embodiment 38, wherein the first polypeptide of the first antigen-binding portion is operably linked at its C-terminus to N-terminus of one of the Fc polypeptides, and the second antigen-binding portion comprises a Fab and a Fab heavy chain of the second antigen-binding portion is operably linked at its C-terminus to N-terminus of the other Fc polypeptide.
Embodiment 40. The bispecific antibody according to any one of Embodiments 32 to 36, further comprising an Fc domain composed of two Fc polypeptides capable of stable association.
Embodiment 41. The bispecific antibody according to Embodiment 40, wherein the first antigen-binding portion is operably linked at its C-terminus to N-terminus of one of the Fc polypeptides, the second antigen-binding portion is operably linked at its C-terminus to N-terminus of the other Fc polypeptide, and the third antigen-binding portion is operably linked at its C-terminus to N-terminus of the first antigen-binding portion or N-terminus of the second antigen-binding portion.
Embodiment 42. The bispecific antibody according to Embodiment 40, wherein the third antigen-binding portion is operably linked at its C-terminus to N-terminus of one of the Fc polypeptides, the first antigen-binding portion is operably linked at its C-terminus to N-terminus of the third antigen-binding portion, and the second antigen-binding portion is operably linked at its C-terminus to N-terminus of the other Fc polypeptide.
Embodiment 43. The bispecific antibody according to Embodiment 40, wherein the third antigen-binding portion is operably linked at its C-terminus to N-terminus of one of the Fc polypeptides, the second antigen-binding portion is operably linked at its C-terminus to N-terminus of the third antigen-binding portion, and the first antigen-binding portion is operably linked at its C-terminus to N-terminus of the other Fc polypeptide.
Embodiment 44. The bispecific antibody according to Embodiments 41 to 43, wherein the third antigen-binding portion is operably linked to the first antigen-binding portion by a peptide linker, or the third antigen-binding portion is operably linked to the second antigen-binding portion by a peptide linker.
Embodiment 45. The bispecific antibody according to any one of Embodiments 37 to 44, wherein the Fc domain is an IgG Fc domain.
Embodiment 46. The bispecific antibody according to Embodiment 45, wherein the IgG Fc domain is a human IgG Fc domain, preferably an Fc domain of human IgG1 or human IgG4.
Embodiment 47. The bispecific antibody according to any one of Embodiments 37 to 46, wherein the two Fc polypeptides are stably associated by one or more of a linker, a disulfide bond, a hydrogen bond, an electrostatic interaction, a salt bridge, and a hydrophobic-hydrophilic interaction.
Embodiment 48. The bispecific antibody according to any one of Embodiments 37 to 47, wherein the Fc domain comprises a modification that promotes association of the two Fc polypeptides of the Fc domain.
Embodiment 49. The bispecific antibody according to any one of Embodiments 37 to 48, wherein one Fc polypeptide comprises T366Y/W and S354C, and the other Fc polypeptide comprises Y407T/V, Y349C, T366S, and L368A according to the EU numbering.
Embodiment 50. The bispecific antibody according to any one of Embodiments 37 to 49, wherein the Fc domain comprises a modification that reduces or eliminates the binding of a CH3 region of one Fc polypeptide in the Fc to Protein A.
Embodiment 51. The bispecific antibody according to any one of Embodiments 37 to 50, wherein according to the EU numbering, the Fc domain comprises an amino acid substitution H435R or/and Y436F, which occurs only in one of the Fc polypeptides.
Embodiment 52. An isolated polynucleotide encoding the bispecific antibody according to any one of Embodiments 1 to 51.
Embodiment 53. An isolated vector comprising the polynucleotide according to Embodiment 52.
Embodiment 54. A host cell comprising the isolated polynucleotide according to Embodiment 52 or the isolated vector according to Embodiment 51.
Embodiment 55. A method of expressing the bispecific antibody according to any one of Embodiments 1 to 51, comprising culturing the host cell according to Embodiment 52 under such conditions that the bispecific antibody is expressed.
Embodiment 56. A pharmaceutical composition comprising the bispecific antibody according to any one of Embodiments 1 to 51 and a pharmaceutically acceptable carrier.
Embodiment 57. A conjugate comprising the bispecific antibody according to any one of Embodiments 1 to 51 and a therapeutic agent linked or conjugated to the bispecific antibody.
Embodiment 58. A fusion protein comprising a fusion component expressed by fusion and the bispecific antibody according to any one of Embodiments 1 to 51.
Embodiment 59. A method of treating a disease in a subject in need thereof, comprising administering to the subject a therapeutically effective amount of the bispecific antibody according to any one of Embodiments 1 to 51, the pharmaceutical composition according to Embodiment 56, the conjugate according to Embodiment 57, or the fusion protein according to Embodiment 58.

### DETAILED DESCRIPTION

### Example 1. Design of MHC-I Element Sequences

According to IPD-IMGT/HLA (https://www.ebi.ac.uk/ipd/imgt/hla/stats.html) database (version 3.42) statistics (Table 1), HLA-I had a larger number of alleles and a wider distribution relative to HLA-II, such that HLA-I molecules were selected to design structural elements of bispecific antibodies to reduce the risk of immunogenicity.

According to IPD-IMGT/HLA database (version 3.42) statistics (Table 2), the number of HLA-I-B alleles was the largest among HLA-I molecules, and thus HLA-I-B molecules were selected to design structural elements of bispecific antibodies to further reduce the risk of immunogenicity.

**Table 1. Number of HLA alleles**

| Allele | Number |
|---|---|
| HLA-I | 20,597 |
| HLA-II | 7,723 |
| HLA | 28,320 |

**Table 2. HLA-I alleles and proteins**

| Type | A | B | C | E | F | G |
|---|---|---|---|---|---|---|
| Alleles | 6,291 | 7,562 | 6,223 | 256 | 45 | 82 |
| Proteins | 3,896 | 4,803 | 3,618 | 110 | 6 | 22 |
| Nulls | 325 | 253 | 272 | 7 | 0 | 4 |

### 1.1. Determination of initial sequences of MHC-I element

In the Uniprot database (https://www.uniprot.org/), a series of α chain full-length sequences of natural HLA-I-B molecules were obtained (Table 3); in the Uniprot database, a β chain (also referred to as β microglobulin) full-length sequence of HLA-I molecules was obtained (Uniprot ID: P61769). Crystal data information on proteins containing α chain of an HLA-I-B molecule was further obtained by a cross-indexing system on the Uniprot database page, and high-resolution crystal structures (Resolution < 2 Å) were selected in order to ensure the accuracy of the crystal data (Table 4).

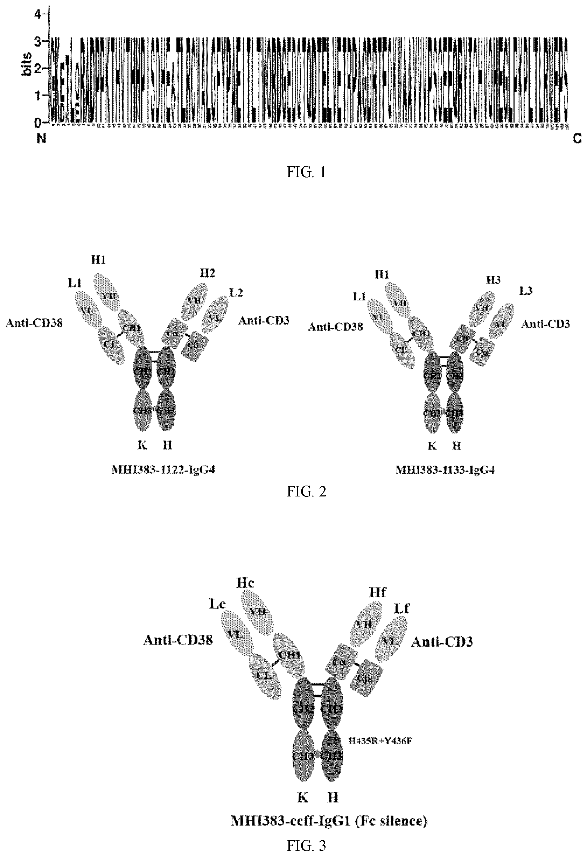

The relative starting positions of α chain constant regions (defined herein as MHCICα, constant region alpha of MHC I, or simply Cα) of a natural HLA-I-B molecule were determined by sequence analysis in combination with information on these crystal structures. There was a certain sequence diversity in the α chain constant region of the HLA-I-B molecule. The sequences in Table 3 were subjected to multi-sequence alignment using an on-line alignment tool ClustalW2 (https://www.ebi.ac.uk/Tools/phylogeny/simple_phylogeny/), and then the alignment results were analyzed and displayed by webLogo (http://weblogo.berkeley.edu/), A consensus sequence of the α chain constant region of the HLA-I-B molecule (FIG. 1) was obtained according to the frequency of occurrence of amino acids at each position, and the consensus sequence of the α chain constant region of the HLA-I-B molecule was used as an initial sequence for subsequent operation and modification. Since there was no sequence diversity in human β2 microglobulin, a sequence of a paired region (defined herein as MHCICβ, constant region beta of MHC I, or simply Cβ) of natural β2 microglobulin with MHCICα was determined by sequence analysis (Uniprot ID: P61769).

Initial sequences of the MHC-I element were as follows:
> MHCICαori (SEQ ID NO: 1) 
GKETLQRADPPKTHVTHHPISDHEATLRCWALGFYPAEITLTWQRDGEDQTQDTELVETRP
AGDRTFQKWAAVVVPSGEEQRYTCHVQHEGLPKPLTLRWEPS
> MHCICβori (SEQ ID NO: 2)

IQRTPKIQVYSRHPAENGKSNFLNCYVSGFHPSDIEVDLLKNGERIEKVEHSDLSFSKDWSF
YLLYYTEFTPTEKDEYACRVNHVTLSQPKIVKWDR

**Table 3. Sequence information on α chain of natural HLA-I-B molecule contained in the Uniprot database**

| No. | Uniprot accession No. |
|---|---|
| 1 | A0A140T9S9 |
| 2 | A0A140T9G0 |
| 3 | A0A140T9H3 |
| 4 | A0A140T9A9 |
| 5 | A0A140T951 |
| 6 | A0A1W2PPR8 |
| 7 | D9J307 |
| 8 | S6AU73 |
| 9 | Q2L6G2 |
| 10 | D5H3J5 |
| 11 | Q5SS57 |
| 12 | P30685 |
| 13 | P01889 |

**Table 4. Crystal structures of proteins containing α chain of HLA-I-B molecule**

| No. | PDB accession No. | Resolution (Å) | No. | PDB accession No. | Resolution (Å) | No. | PDB accession No. | Resolution (Å) | No. | PDB accession No. | Resolution (Å) |
|---|---|---|---|---|---|---|---|---|---|---|---|
| 1 | 1K5N | 1.09 | 31 | 6PYL | 1.52 | 61 | 1N2R | 1.7 | 91 | 4O2C | 1.8 |
| 2 | 4U1M | 1.18 | 32 | 6PZ5 | 1.53 | 62 | 1SYV | 1.7 | 92 | 4PR5 | 1.8 |
| 3 | 3CZF | 1.2 | 33 | lUXS | 1.55 | 63 | 1ZSD | 1.7 | 93 | 5IEK | 1.8 |
| 4 | 6MT3 | 1.21 | 34 | 3VFV | 1.55 | 64 | 2NW3 | 1.7 | 94 | SVUE | 1.8 |
| 5 | 3BWA | 1.3 | 35 | 6MT4 | 1.55 | 65 | 3DX6 | 1.7 | 95 | 5VWD | 1.8 |
| 6 | 3LN4 | 1.3 | 36 | 6MT5 | 1.55 | 66 | 3L3I | 1.7 | 96 | SVWF | 1.8 |
| 7 | 3SPV | 1.3 | 37 | 5WMR | 1.58 | 67 | 3VCL | 1.7 | 97 | 5WMN | 1.82 |
| 8 | 6MT6 | 1.31 | 38 | 6BXQ | 1.58 | 68 | 4QRQ | 1.7 | 98 | SDEG | 1.83 |
| 9 | 2BVP | 1.35 | 39 | 4U1H | 1.59 | 69 | 5EO0 | 1.7 | 99 | 6D2R | 1.83 |
| 10 | 6MTL | 1.35 | 40 | 6P23 | 1.59 | 70 | STXS | 1.7 | 100 | 3KPQ | 1.84 |
| 11 | 4U1J | 1.38 | 41 | 6P27 | 1.59 | 71 | 5XOS | 1.7 | 101 | 3BP4 | 1.85 |
| 12 | 6PYW | 1.38 | 42 | 1M6O | 1.6 | 72 | lUXW | 1.71 | 102 | 3VFS | 1.85 |
| 13 | 2A83 | 1.4 | 43 | 1ZHK | 1.6 | 73 | 3D18 | 1.74 | 103 | 5EO1 | 1.85 |
| 14 | 4QRS | 1.4 | 44 | 3DX7 | 1.6 | 74 | 3BW9 | 1.75 | 104 | 3B3I | 1.86 |
| 15 | 4QRT | 1.4 | 45 | 3KPM | 1.6 | 75 | 6BJ8 | 1.75 | 105 | 3C9N | 1.87 |
| 16 | 5WMQ | 1.4 | 46 | 3SKO | 1.6 | 76 | 4U1S | 1.76 | 106 | 6D29 | 1.88 |
| 17 | 6P2C | 1.4 | 47 | 3VRI | 1.6 | 77 | 5T6Y | 1.76 | 107 | 1JGD | 1.9 |
| 18 | 4XXC | 1.43 | 48 | 4G8I | 1.6 | 78 | 4U1N | 1.77 | 108 | 1M05 | 1.9 |
| 19 | SIB2 | 1.44 | 49 | 4G9D | 1.6 | 79 | 1XR9 | 1.79 | 109 | 2H6P | 1.9 |
| 20 | 6PYJ | 1.44 | 50 | 4LCY | 1.6 | 80 | 2AXF | 1.8 | 110 | 3KPP | 1.9 |
| 21 | 6BXP | 1.45 | 51 | 4QRU | 1.6 | 81 | 3B6S | 1.8 | ill | 3LKS | 1.9 |
| 22 | 6PYV | 1.45 | 52 | 5DEF | 1.6 | 82 | 3BP7 | 1.8 | 112 | 3LN5 | 1.9 |
| 23 | 1OGT | 1.47 | 53 | 5WMP | 1.6 | 83 | 3L3D | 1.8 | 113 | 3VRJ | 1.9 |
| 24 | 1XH3 | 1.48 | 54 | 5WMO | 1.62 | 84 | 3LKO | 1.8 | 114 | 4G9F | 1.9 |
| 25 | 6P2F | 1.48 | 55 | 2BVO | 1.65 | 85 | 3LKP | 1.8 | 115 | 4JQX | 1.9 |
| 26 | 1ZHL | 1.5 | 56 | 3VFU | 1.65 | 86 | 3LKQ | 1.8 | 116 | 4O2F | 1.9 |
| 27 | 2HJL | 1.5 | 57 | 4PRN | 1.65 | 87 | 3SKM | 1.8 | 117 | ST6W | 1.9 |
| 28 | 4JQV | 1.5 | 58 | SVWH | 1.65 | 88 | 3VH8 | 1.8 | 118 | 5VUF | 1.9 |
| 29 | 5IEH | 1.5 | 59 | 6P2S | 1.65 | 89 | 3X12 | 1.8 | 119 | 6BJ3 | 1.9 |
| 30 | 6AT5 | 1.5 | 60 | 5T6X | 1.69 | 90 | 3X13 | 1.8 | 120 | 6D2T | 1.9 |

### 1.2. Optimization of initial sequences of MHC-I element

### 1.2.1. Introduction of interchain disulfide bonds to enhance pairing stability of MHC-I element

The amino acid sequences of MHCICαori and MHCICβori described above were loaded into Molecular Operating Environment (MOE) software, and heterodimer modeling was performed on the sequences through Homology modeling module of MOE, so that a simulation structure of the MHC-I element was obtained. In the natural state, no disulfide bonds exist between the constant regions of MHC molecules. To improve the pairing stability of the constant regions of MHC-I and optimize its developability, cysteine (Cys) could be introduced at position R60, A62, or G63 for MHCICαori, and cysteine (Cys) could be introduced at position Y26, R12, or Y67 for MHCICβori, at the contact interface of the domains of MHCICα and MHCICβ. Delta stability after the introduction of disulfide bonds was simulated and calculated through Disulfide scan module of MOE, wherein when A62C mutation was introduced into MHCICαori and R12C mutation was introduced into MHCICβori, there was the lowest delta stability of -1.62 kcal/mol, indicating that the mutations at these two positions can form the most stable disulfide bond at the interface of Cα and Cβ (Table 5).

**Table 5. Simulation results of disulfide bond introduction at interface of MHCICαori and MHCICβori**

| MHCICαori | MHCICβori | delta Stability (kcal/mol) |
|---|---|---|
| R60C | Y26C | 1.96 |
| A62C | R12C | -1.62 |
| G63C | Y67C | 1.86 |

### 1.2.2. Isoelectric point optimization

The theoretical isoelectric point (pI) of the MHC-I element after the introduction of disulfide bonds (A62C and R12C in Table 5) into the initial sequences was 5.80, which was not conducive to subsequent process development. Thus, we could modify the related charges of the MHC-I element to improve the druggability. Solvent accessible surface (SAS) and charged amino acid residues were analyzed for the MHC-I element through Protein-properties module of MOE software. In the MHC-I element, amino acid residues with solvent exposure degrees greater than 40% and exhibiting negative charge/acidity are shown in Table 6. Considering the structural environment, chemical environment and application purposes of the related residues at the same time, one or more of these acidic amino acid residues were substituted with a basic amino acid residue (arginine (R) or lysine (K)), thereby increasing the theoretical isoelectric point of the MHC-I element to 6.5-9.0. In a specific embodiment, mutations D22R, E48K, and D53K at three sites were introduced in MHCICα, and a single-point mutation E69R was introduced in MHCICβ, thereby increasing the theoretical isoelectric point of the MHC-I element to 7.8.

**Table 6. SAS and residue charge properties of molecules in MHC-I element**

| Subunit sequence | Amino acid residue | Position | Acid-base property | Solvent exposure degree (%) | Acid dissociation constant pKa |
|---|---|---|---|---|---|
| MHCICαori | ASP | 22 | Acidic | 85.17 | 3.19 |
| MHCICαori | GLU | 48 | Acidic | 67.65 | 3.54 |
| MHCICαori | GLU | 3 | Acidic | 53.81 | 3.72 |
| MHCICαori | GLU | 90 | Acidic | 51.81 | 4.78 |
| MHCICαori | GLU | 101 | Acidic | 44.6 | 2.56 |
| MHCICαori | ASP | 53 | Acidic | 43.3 | 3.37 |
| MHCICβori | GLU | 74 | Acidic | 67.85 | 3.91 |
| MHCICβori | GLU | 47 | Acidic | 64.08 | 3.17 |
| MHCICβori | GLU | 69 | Acidic | 49.51 | 3.84 |
| MHCICβori | ASP | 34 | Acidic | 48.31 | 3.1 |
| MHCICβori | GLU | 16 | Acidic | 47.83 | 3.65 |
| MHCICβori | ASP | 53 | Acidic | 47.27 | 2.31 |
| MHCICβori | GLU | 44 | Acidic | 44.71 | 2.32 |
| MHCICβori | GLU | 50 | Acidic | 41.72 | 3.98 |
| MHCICβori | GLU | 36 | Acidic | 40.96 | 2.81 |

### 1.2.3. Other optimizations

Considering the 4 amino acids (-KWDR) at the end of the Cβ region, their side chains are relatively large, which can cause potential steric hindrance effects. The stability of the protein conformation will be reduced if a flexible G4S linker peptide is used. The structural characteristics of glycine (G) and proline (P) determine that the two amino acids are mainly located at random coil or corner positions. Glycine has no side chain group, which can well alleviate the steric hindrance effect, while the side chain of proline is a five-membered ring structure and has certain rigidity, which can improve conformation stability, so that GP was finally added at the carboxyl-terminus of the Cβ region to balance the potential steric hindrance effects and conformation stability.

Optimized sequences of the MHC-I element were as follows:
> Cα (SEQ ID NO: 3) 
GKETLQRADPPKTHVTHHPISRHEATLRCWALGFYPAEITLTWQRDGKDQTQKTELVETR
PCGDRTFQKWAAVVVPSGEEQRYTCHVQHEGLPKPLTLRWEPS
> Cβ (SEQ ID NO: 4)

IQRTPKIQVYSCHPAENGKSNFLNCYVSGFHPSDIEVDLLKNGERIEKVEHSDLSFSKDWSF
YLLYYTRFTPTEKDEYACRVNHVTLSQPKIVKWDRGP

### Example 2. Construction and Characterization of MHC-I Element-Modified Bispecific IgG4 Antibodies

To ensure that the MHC-I element could be used for bispecific antibodies, daratumumab and human-targeting CD3 antibody SP34 were selected to construct bispecific antibodies targeting CD3 and CD38 for early proof of concepts and to investigate the effect of the relative orientations of Cα and Cβ on MHC-I element-modified antigen-binding portions. To avoid or alleviate the mispairing between IgG heavy chains, bispecific antibodies subsequently constructed both adopted a "knobs into holes" structure. The IgG constant region domain CH1/CL of SP34 Fab was replaced by the corresponding Cα/Cβ and Cβ/Cα, respectively, to construct MHC-I-modified antigen-binding portions with different relative orientations, and the heavy chain of the SP34 arm adopted a "hole" structure, i.e., CH3-hole. The Fab of daratumumab remained unchanged, and its heavy chain adopted a "knob" structure, i.e., CH3-knob. The MHC-I-modified antigen-binding portions targeting CD3 were combined with the Fab of daratumumab to create two different bispecific antibodies, named MHI383-1122-IgG4 and MHI383-1133-IgG4, respectively (molecular structures shown in FIG. 2), with the sequences shown in Table 7. The MHC-I elements adopted Cα of the sequence set forth in SEQ ID NO: 3 and Cβ of the sequence set forth in SEQ ID NO: 4.

**Table 7. Sequences of bispecific antibodies MHI383-1122-IgG4 and MHI383-1133-IgG4**

| Name | Polypeptide chain compositions | Amino acid sequence | Nucleotide sequence |
|---|---|---|---|
| MHI383-1122-IgG4 | H1 | SEQ ID NO: 6 | SEQ ID NO: 7 |
| | L1 | SEQ ID NO: 8 | SEQ ID NO: 9 |
| | H2 | SEQ ID NO: 10 | SEQ ID NO: 11 |
| | L2 | SEQ ID NO: 12 | SEQ ID NO: 13 |
| MHI383-1133-IgG4 | H1 | SEQ ID NO: 6 | SEQ ID NO: 7 |
| | L1 | SEQ ID NO: 8 | SEQ ID NO: 9 |
| | H3 | SEQ ID NO: 14 | SEQ ID NO: 15 |
| | L3 | SEQ ID NO: 16 | SEQ ID NO: 17 |

For each bispecific antibody, DNA sequences of each polypeptide chain for encoding and constituting the antibody were inserted into vectors, respectively, to obtain expression vectors expressing the corresponding polypeptide chains. 25 µg of the expression vectors encoding the polypeptide chains of each multispecific antibody were co-transfected into ExpiCHO-S cells (manufacturer: Shanghai Institute of Pharmaceutical Industry, Cat No. 127200005) at a transfection expression volume of 100 mL and a cell density of 6E+06 cells/mL.

After transfection, the cells were expressed and cultured at 32 °C with 5% CO₂ at 130 rpm. On day 8, the cell supernatant was collected, and the antibody protein was purified in one step by Protein A magnetic beads (manufacturer: GenScript, Cat No. L00695-20). 4 mL of magnetic beads were added to the cell supernatant, and the mixture was added to a 50 mL centrifuge tube. The centrifuge tube was placed in a rotary decolorization shaker (manufacturer: Shanghai ZHICHENG, model: ZHWY-304), and the mixture was incubated at room temperature for 2 h. The magnetic beads were adsorbed on the base of a magnetic rack, the supernatant was discarded, and the magnetic beads were washed thoroughly with a buffer. The antibody proteins were eluted with 0.1 mol/L glycine with a pH of 3.0 (manufacturer: Sinopharm, Cat No. 62011516). The eluate was placed in an ultrafiltration tube (manufacturer: Millipore, Cat No. UFC501096), concentrated, subjected to buffer exchange with 1×PBS (manufacturer: Shanghai Sangon, Cat No. B040100-0005), and purified to obtain MHI383-1122-IgG4 and MHI383-1133-IgG4.

### 2.1. Detection of binding affinity of bispecific antibodies to antigens by BLI

Human CD38 protein (Beijing ACROBiosystems, Cat No. CD8-H5224) and human CD3E (Beijing ACROBiosystems, Cat No. CDE-H5223) were used as antigens. The affinity of MHI383-1122-IgG4 and MHI383-1133-IgG4 to human CD38 and CD3E proteins was separately assayed by bio-layer interferometry (BLI) technique. The specific experimental procedures were as follows:
The affinity assays were used to evaluate the affinity of MHI383-1122-IgG4 and MHI383-1133-IgG4 to human CD38 and CD3E proteins. The affinity assays were mainly performed by BLI technique. MHI383-1122-IgG4 or MHI383-1133-IgG4 were separately immobilized on a sensor, and then the sensor was immersed in human CD38 and CD3E protein solutions with certain concentration gradients, respectively. Association/dissociation curves were acquired in real time by Data Acquisition 11.0.0.64, and data analysis was performed by Data Analysis 11.0. An association rate constant Ka, a dissociation rate constant Kd, and an equilibrium constant KD were obtained, the equilibrium constant KD representing the affinity.

**Table 8. Affinity of MHI383-1122-IG4 and MHI383-1133-IgG4 (BLD**

| Sample | Antigen | kon(1/Ms) | kdis(1/s) | KD (M) | Antigen | kon(1/Ms) | kdis(1/s) | KD (M) |
|---|---|---|---|---|---|---|---|---|
| MHI383-1122-IgG4 | human CD3E | 2.05E+05 | 2.46E-04 | 1.20E-09 | human CD38 | 2.17E+05 | 3.69E-03 | 1.70E-08 |
| MHI383-1133-IgG4 | human CD3E | 1.43E+05 | 9.17E-05 | 6.41E-10 | human CD38 | 2.94E+05 | 1.80E-03 | 6.14E-09 |

The experimental results showed that the bispecific antibodies MHI383-1122-IgG4 and MHI383-1133-IgG4 with different Cα/Cβ orientations both showed good affinity to human CD38 and CD3E proteins, and the difference in affinity KD values between them was less than 3 times, which was mainly caused by the loss of their dissociation rate constants Kd (Table 8). Thus, the MHC-I elements (Cα and Cβ) could replace the CH1 and CL domains for the construction of bispecific antibodies.

### Example 3. Construction and Characterization of MHC-I Element-Modified Bispecific IgG1 Antibodies

To avoid or alleviate the mispairing between IgG heavy chains, bispecific antibodies constructed both adopted a "knobs into holes" structure. To test the effect of MHC-I elements (Cα of the sequence set forth in SEQ ID NO: 3 and Cβ of the sequence set forth in SEQ ID NO: 4) on bispecific antibodies of different IgG subtypes, we engineered the MHI383-1122-IgG4 described above into an IgG1 form, creating a novel bispecific antibody named MHI383-ccff-IgG1 (construction structure as shown in FIG. 3). The CH2 domains of two heavy chains of this antibody adopted LALA (L234A + L235A) double mutation to eliminate the potential Fc effector effect. In this example, H435R + Y436F (EU numbering) double mutation was introduced into the CH3 domain of the MHC-I element-modified heavy chain. A heavy chain-mispaired product containing the H435R + Y436F double mutation was not combined with protein A, so that the heavy chain-mispaired product was removed in protein A affinity chromatography purification, thereby improving the purity and purification efficiency of the bispecific antibody of interest.

MHI383-ccff-IgG1 has four polypeptide chains (Hc, Lc, Hf, and Lf) with the following sequences:
He: an amino acid sequence is set forth in SEQ ID NO: 18, and a nucleotide sequence is set forth in SEQ ID NO: 19;
Lc: an amino acid sequence is set forth in SEQ ID NO: 20, and a nucleotide sequence is set forth in SEQ ID NO: 21;
Hf: an amino acid sequence is set forth in SEQ ID NO: 22, and a nucleotide sequence is set forth in SEQ ID NO: 23; and
Lf: an amino acid sequence is set forth in SEQ ID NO: 24, and a nucleotide sequence is set forth in SEQ ID NO: 25.

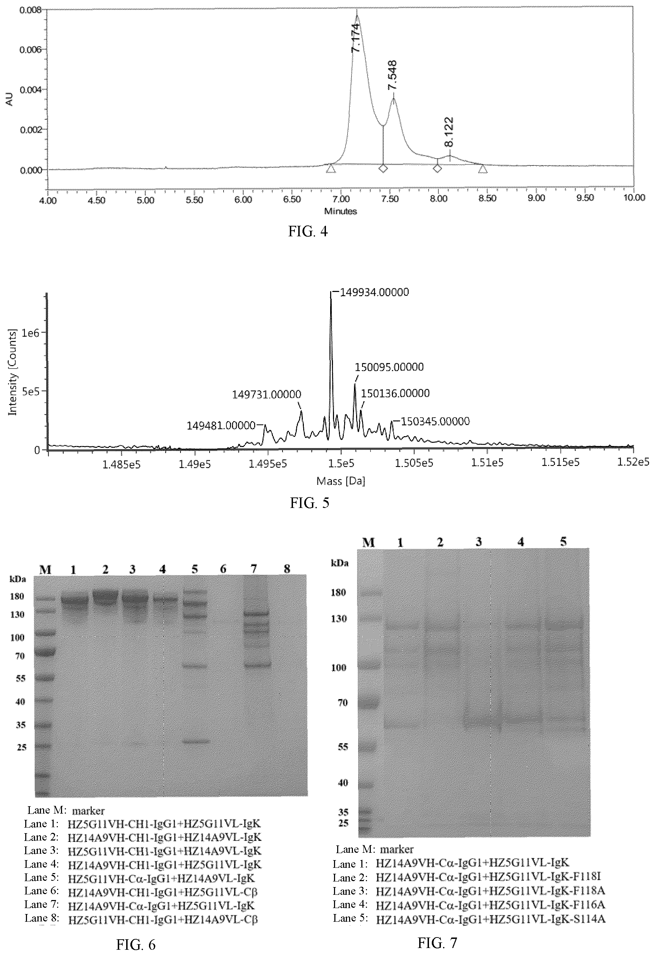

The eluted target protein peak was further purified by preparative size exclusion chromatography using SuperdexTM 200 Increase 10/300GL column and AKTA system from GE. The purification experiment was performed using an equilibration buffer (50 mM Tris-HAc, 150 mM L-Arg-HCl, pH 7.5) at a flow rate of 0.8 mL/min. FIG. 4 is an SEC purification map of an MHI383-ccff-IgG1 molecule. The SEC main peak (retention time RT = 7.174 min) was collected for subsequent mass spectrometry analysis. A complete molecular weight identification was performed on the SEC main peak described above by liquid chromatography-mass spectrometry.

The complete molecular weight analysis was performed using a Waters LC-MS system (Waters, Singapore, USA, UK). A chromatography column MAbPac RP 4 µm 2.1 × 50 mm (Thermo, USA) was adopted. Phase A (0.1% aqueous formic acid solution) and phase B (0.1% formic acid in acetonitrile) were used as mobile phases, and the detection wavelength was 280 nm. 1 µg of protein was injected into the liquid chromatography-mass spectrometry system with a gradient of 5% B to 100% B within 5.5 min. The mass spectrometer adopted a positive ion mode, and the scanning range was 200-4000 m/z. Data were collected using MassLynx 4.1 and processed by UNIFI 1.8.2.169. The mass spectrometry results showed that the peak at 149934 Da (FIG. 5) was consistent with the expected complete molecular weight of the correctly assembled bispecific antibody MHI383-ccff-IgG1.

Based on sequence pattern analysis of the amino acid sequences of the MHC-I elements, we did not find N-linked glycosylation site motifs (NXS/T, X = any amino acid other than proline). However, O-linked glycosylation sites were difficult to be predicted from the sequences. Thus, the N-glycan present in the SEC main peak protein described above was further cleaved by hydrolysis using the glycosidase PNGaseF. The protein after N-glycan cleavage was subjected to peptide mapping characterization (protein sequencing) by tandem mass spectrometry to investigate whether there were possible O-glycosylation sites.

Peptide sequence coverage analysis was performed using a Waters LC-MS system (Waters, Singapore, USA, UK). A chromatography column AdvanceBio Peptide Map 2.1 × 150 mm 2.7-Micron (Aglient, USA) was adopted. Phase A (0.1% aqueous formic acid solution) and phase B (0.1% formic acid in acetonitrile) were used as mobile phases, and the detection wavelength was 214 nm. The protein after PNGaseF treatment was denatured, reduced and alkylated, and then the protein was digested by trypsin overnight. A proper amount of the protein after digestion was injected into the liquid chromatography-mass spectrometry system for detection. The elution gradient was 0% B to 15% B within 13 min, and 15% B to 40% B within 45 min. The mass spectrometer adopted a positive ion mode, and the scanning range was 100-2000 m/z. Data were collected using MassLynx 4.1 and processed by UNIFI 1.8.2.169.

The peptide mapping experiment results also showed that there was no O-linked glycosylation modification in the MHI383-ccff-IgG1 molecule, thereby indicating that there is also no O-linked glycosylation site in the MHC-I element. Compared with the bispecific antibody based on TCR modification, the MHC-I element-modified bispecific antibodies have a lower level of glycoform heterogeneity and are more favorable for downstream process development and quality control.

### Example 4. Design of Mutation Sites and Chain Mispairing Verification

The antibody HZ5G11 is targeted to the extracellular domain of human PD-L1 protein, and the antibody HZ14A9 is targeted to the extracellular domain of human CD47 protein. Both antibodies are monoclonal antibodies obtained by hybridoma screening and performing humanization after immunizing mice with corresponding antigens.

The monoclonal IgGIK antibody HZ5G11 targeting human PD-L1 consists of a heavy chain HZ5G11VH-CH1-IgG1 (with a heavy chain amino acid sequence set forth in SEQ ID NO: 26 and a nucleotide sequence set forth in SEQ ID NO: 27) and a light chain HZ5G11VL-IgK (with a light chain amino acid sequence set forth in SEQ ID NO: 28 and a nucleotide sequence set forth in SEQ ID NO: 29). The monoclonal IgGIK antibody HZ14A9 targeting human CD47 consists of a heavy chain HZ14A9VH-CH1-IgG1 (with a heavy chain amino acid sequence set forth in SEQ ID NO: 45) and a light chain HZ14A9VL-IgK (with a light chain amino acid sequence set forth in SEQ ID NO: 46). HZ14A9- and HZ5G11-related variants based on MHC-I elements were further constructed as follows: the IgG1 constant region domain CH1 of the heavy chain of the HZ14A9 antibody was replaced by MHCICα (SEQ ID NO: 3) in the corresponding MHC-I element to create a heavy chain HZ14A9VH-Cα-IgG1 (with a heavy chain amino acid sequence set forth in SEQ ID NO: 47), and the IgK constant region domain CL of the light chain of the HZ14A9 antibody was replaced by MHCICβ (SEQ ID NO: 4) in the corresponding MHC-I element to create a light chain HZ14A9VL-Cβ (with a light chain amino acid sequence set forth in SEQ ID NO: 48); the IgG1 constant region domain CH1 of the heavy chain of the HZ5G11 antibody was replaced by MHCICα in the corresponding MHC-I element to create a heavy chain HZ5G11VH-Cα-IgG1 (with a heavy chain amino acid sequence set forth in SEQ ID NO: 30 and a nucleotide sequence set forth in SEQ ID NO: 31), and the IgK constant region domain CL of the light chain of the HZ5G11 antibody was replaced by MHCICβ in the corresponding MHC-I constant region element to create a light chain HZ5G11VL-Cβ (with a light chain amino acid sequence set forth in SEQ ID NO: 32 and a nucleotide sequence set forth in SEQ ID NO: 33).

To verify whether there was a possible interaction between the MHC-I elements MHCICα and MHCICβ with the antibody constant region CH1 and light chain constant region CL, plasmids encoding different heavy chains or light chains were constructed separately, combined and then purified for expression in pairs as shown in Table 9. Specifically, DNA sequences were synthesized, and the DNA sequences encoding the heavy chain and light chain were inserted into vectors (e.g., pcDNA3.1 vector disclosed in CN107001463A, pCHO1.0 vector disclosed in CN109422811A, etc.), respectively, to obtain recombinant expression vectors expressing the corresponding heavy chain or light chain. According to a mass ratio of 1:1, the recombinant expression vectors expressing the heavy chain and the light chain were co-transfected into ExpiCHO-S cells (manufacturer: Shanghai Institute of Pharmaceutical Industry, Cat No. 127200005) at a transfection expression volume of 0.1-1 L and a cell density of 6E+06 cells/mL.

After transfection, the cells were placed in ExpiCHO^{™} medium (manufacturer: Thermo, Cat No. A2910001), and expressed and cultured at 32 °C with 5% CO₂ at 130 rpm. On day 10, the cell supernatant was collected, and the target protein was purified by protein A affinity chromatography using AKTA Pure 25 L system from GE and MabSelect SuRe LX packing. After elution, the concentration of the eluted protein was measured using NanoDrop Lite (Thermo Fisher Scientific). The purity of the eluted protein was measured by SEC-HPLC, and the protein purity of the sample used for the test was not lower than 80%. A total amount of 5 mg of protein was taken from each sample and subjected to non-reducing SDS-PAGE electrophoresis. The protein was stained with Coomassie brilliant blue, and the electrophoresis results were observed.

**Table 9. Combinations of different heavy chain/light chain expression in pairs**

| Combination | Heavy chain | Light chain |
|---|---|---|
| 1 | HZ5G11VH-CH1-IgG1 | HZ5G11VL-IgK |
| 2 | HZ14A9VH-CH1-IgG1 | HZ14A9VL-IgK |
| 3 | HZ5G11VH-CH1-IgG1 | HZ14A9VL-IgK |
| 4 | HZ14A9VH-CH1-IgG1 | HZ5G11VL-IgK |
| 5 | HZ5G11VH-Cα-IgG1 | HZ14A9VL-IgK |
| 6 | HZ14A9VH-CH1-IgG1 | HZ5G11VL-Cβ |
| 7 | HZ14A9VH-Cα-IgG1 | HZ5G11VL-IgK |
| 8 | HZ5G11VH-CH1-IgG1 | HZ14A9VL-Cβ |

As shown in FIG. 6, the experimental results showed that hybrid paired products were still generated based on the exchange of light and heavy chains between the HZ5G11 and HZ14A9 monoclonal antibodies, respectively, and the molecular weights of the hybrid paired products were between those of the HZ5G11 and the HZ14A9 monoclonal antibodies. After the co-expression and purification of HZ5G11VH-Ca-IgG1 and HZ14A9VL-IgK as well as HZ14A9VH-Cα-IgG1 and HZ5G11VL-IgK, various products were generated. Among them, the combination of HZ5G11VH-Cα-IgG1 and HZ14A9VL-IgK showed that the molecular weight of the hybrid paired product was 130-180 kDa, which was close to that of the HZ5G11 and HZ14A9 monoclonal antibodies. While the combination of HZ14A9VH-Cα-IgG1 and HZ5G11VL-IgK showed that the molecular weight of the hybrid paired product was 100-130 kDa, which was easier to distinguish from the HZ5G11 and HZ14A9 monoclonal antibodies. The results above indicate that there is some interaction between the CL domain and MHCICα, thereby promoting the production of light and heavy-mispaired products. After the co-expression and purification of HZ14A9VH-CH1-IgG1 and HZ5G11VL-Cβ as well as HZ5G11VH-CH1-IgG1 and HZ14A9VL-Cβ, no significant band was found in the lanes of the corresponding non-reducing SDS-PAGE, that is, no significant hybrid paired products were generated, indicating that there is no significant interaction between the CH1 domain and MHCICβ.

To solve the problem of the mispairing between the heavy chain containing MHCICα and the light chain containing CL domain, a structural analysis was performed on the contact interface of domains of MHCICα (SEQ ID NO: 3)/MHCICβ (SEQ ID NO: 4) and IgG1 constant region CH1 (SEQ ID NO: 34)/IgK constant region CL (SEQ ID NO: 35). Combined with a sequence analysis, we believe that 114 (EU numbering), 116 (EU numbering), and 118 (EU numbering) sites in the CL domain may be involved in the interchange process of MHCICβ and the CL domain in subsequent bispecific antibody assembly, thereby generating an interchange mispaired product. To verify the effect of single-point mutations at S114 (EU numbering), F116 (EU numbering), and F118 (EU numbering) in the CL domain of the IgK constant region on the pairing process of MHCICα and the CL domain, non-aromatic hydrophobic amino acids (M: methionine, A: alanine, V: valine, I: isoleucine, or L: leucine) were introduced at the S114 (EU numbering), F116 (EU numbering), or F118 (EU numbering) site in the CL domain of the light chain HZ5G11VL-IgK, respectively, and expressed in combination with the heavy chain HZ14A9VH-Cα-IgG1. The sequence information of several constructed antibody light chains containing mutations is shown in Table 10, and several combinations of different heavy chain/light chain expression in pairs are shown in Table 11.

**Table 10. Sequence information of several constructed antibody light chains containing mutations**

| Plasmid | Amino acid sequence | Nucleotide sequence |
|---|---|---|
| HZ5G11VL-IgK-S114A | SEQ ID NO: 36 | SEQ ID NO: 37 |
| HZ5G11VL-IgK-F116A | SEQ ID NO: 38 | SEQ ID NO: 39 |
| HZ5G11VL-IgK-F118A | SEQ ID NO: 40 | SEQ ID NO: 41 |
| HZSG11VL-IgK-F118I | SEQ ID NO: 42 | SEQ ID NO: 43 |

**Table 11. Several combinations of different heavy chain/light chain expression in pairs**

| Combination | Light chain | Heavy chain |
|---|---|---|
| 1 | HZ5G11VL-IgK | HZ14A9VH-Cα-IgG1 |
| 2 | HZ5G11VL-IgK-S114A | HZ14A9VH-Cα-IgG1 |
| 3 | HZ5G11VL-IgK-F116A | HZ14A9VH-Cα-IgG1 |
| 4 | HZ5G11VL-IgK-F118I | HZ14A9VH-Cα-IgG1 |
| 5 | HZ5G11VL-IgK-F118A | HZ14A9VH-Cα-IgG1 |

In the case of bispecific antibody development, a mispaired product with a molecular weight greater than 100 kDa may not be effectively separated from the product of interest or increase the separation difficulty, thereby affecting the quality of the bispecific antibody product. The results showed that the paired combination of HZSG11VL-IgK-F118A and HZ14A9VH-Cα-IgG1 could significantly reduce the generation of the paired product with a molecular weight greater than 100 kDa (FIG. 7), indicating that the single-point mutation F118A can reduce the acting force between MHCICα and the CL domain, such that the interchange process of MHCICβ and the CL domain in the subsequent bispecific antibody assembly is further solved or alleviated, thereby improving the quality of the bispecific antibody product.

### Example 5. Effect of F118A Mutation in CL Domain on Monoclonal Antibody Assembly

To verify the effect of a single-point mutation F118A (EU numbering) in the CL domain of the IgK constant region on monoclonal antibody assembly, the single-point mutation F118A was separately introduced into the light chain IgK constant regions of monoclonal antibodies targeting CD47 and PD-L1 (HZ14A9 and HZ5G11), and the mutated light chains were expressed in pairs with their original heavy chains, respectively, to obtain HZ14A9-F118A and HZ5G11-F118A. Meanwhile, wild-type monoclonal antibodies targeting CD47 and PD-L1 (HZ14A9 and HZ5G11) were expressed as controls, respectively. Antibody construction and expression methods refer to Example 4.

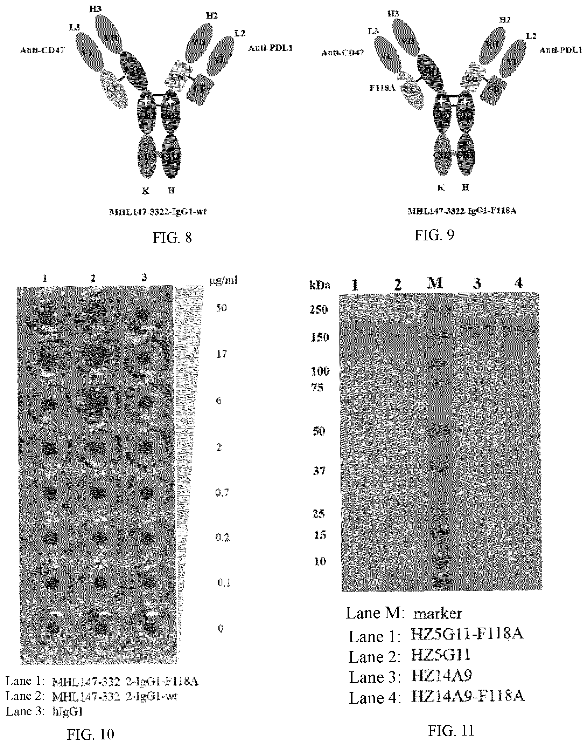

As shown in FIG. 11, the results of the non-reducing SDS-PAGE experiments showed that the bands of all the tested samples were located around 150 kDa. It is indicated that the single-point mutation F118A (EU numbering) in the CL domain of the IgK constant region does not affect the assembly of the monoclonal antibodies.

### Example 6. Construction and Performance Characterization of Anti-PDL1*CD47 Bispecific Antibodies

### 5.1. Construction and expression

To verify the effect of a single-point mutation F118A (EU numbering) in the CL domain of the IgK constant region on bispecific antibodies, two bispecific antibodies targeting CD47 and PD-L1 were constructed, in which the antigen-binding portion targeting PD-L1 was an MHC-I element-modified antigen-binding portion, CH1/CL of the antibody HZ5G11 was replaced by MHCICα /MHCICP, and the antigen-binding portion targeting CD47 was a Fab. Based on the construction of the antibody HZ14A9, the CL domain of the constant region of the Fab light chain was not mutated at F118 (EU numbering) site and carried the single-point mutation F118A. The CH2 domains of two heavy chains of both antibodies adopted LALA (L234A + L235A) double mutation to eliminate the potential Fc effector effect. The CH3 domain carried a KIH mutation to promote heterodimer formation. Thus, two bispecific antibodies targeting CD47 and PD-L1 at the same time were generated, which were MHL147-3322-IgG1-wt (molecular structures shown in FIG. 8) and MHL147-3322-IgG1-F118A (molecular structures shown in FIG. 9), respectively.

**Table 12. Sequences of anti-PDL1*CD47 bispecific antibodies**

| Name of antibody | Polypeptide chain | Amino acid sequence |
|---|---|---|
| MHL147-3322-IgG 1-wt | H2 | (SEQ ID NO: 49) |
| | L2 | (SEQ ID NO: 50) |
| | H3 | (SEQ ID NO: 51) |
| | L3 | DIQMTQSPSSLSASVGDRVTITCKASENVVSYVSWY QQKPGKAPKLLIYGASNRYTGVPSRFIGSGSSTDFTL TISSLQPEDFATYYCGQSYSYPLTFGQGTKLEIK RTVAAPSVFIFPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC (SEQ ID NO: 53) |
| MHL147-3322-F118A | H2 | QITLKESGPTLVKPTQTLTLTCTVSGFSLSTYGVHWIR QPPGKALEWLGVIWRGVTTDYNAAFMSRLTITKDNS KNQVVLTMNNMDPVDTATYYCARLGFYAMDYWGQ GTLVTVSS GKETLQRADPPKTHVTHHPISRHEATLRCWALGFYP AEITLTWQRDGKDQTQKTELVETRPCGDRTFQKWAA VVVPSGEEQRYTCHVQHEGLPKPLTLRWEPS EPKSSDKTHTCPPCPAPEAAGGPSVFLFPPKPKDTLMI SRTPEVTCVVVDVSHEDPEVKFNWYVDGVEVHNAK TKPREEQYNSTYRVVSVLTVLHQDWLNGKEYKCKV SNKALPAPIEKTISKAKGQPREPQVCTLPPSRDELTKN QVSLSCAVKGFYPSDIAVEWESNGQPENNYKTTPPV LDSDGSFFLVSKLTVDKSRWQQGNVFSCSVMHEALH NRFTQKSLSLSPGK (SEQ ID NO: 49) |
| | L2 | DIQMTQSPSSLSASVGDRVTITCKASQSVSNDVAWY QQKPGKAPKLLIYYAANRYTGVPDRFSGSGYGTDFT FTISSLQPEDIATYFCQQDYTSPYTFGQGTKLEIK IQRTPKIQVYSCHPAENGKSNFLNCYVSGFHPSDIEV |
| | | DLLKNGERIEKVEHSDLSFSKDWSFYLLYYTRFTPTE KDEYACRVNHVTLSQPKIVKWDRGP (SEQ ID NO: 50) |
| | H3 | QVQLVQSGAEVKKPGASVKVSCKVSGFNIEDDYIEW VRQAPGQGLEWMGRIDPANDKTKYAQKFQGRVTMT GDTSTNTVYMELSSLRSEDTAVYYCARPGLRRYYSM DYWGQGTLVTVSS ASTKGPSVFPLAPSSKSTSGGTAALGCLVKDYFPEPV TVSWNSGALTSGVHTFPAVLQSSGLYSLSSVVTVPSS SLGTQTYICNVNHKPSNTKVDKKVEPKSCDKTHTCP PCPAPEAAGGPSVFLFPPKPKDTLMISRTPEVTCVVV DVSHEDPEVKFNWYVDGVEVHNAKTKPREEQYNST YRVVSVLTVLHQDWLNGKEYKCKVSNKALPAPIEKT ISKAKGQPREPQVYTLPPCRDELTKNQVSLWCLVKG FYPSDIAVEWESNGQPENNYKTTPPVLDSDGSFFLYS KLTVDKSRWQQGNVFSCSVMHEALHNHYTQKSLSL SPGK (SEQ ID NO: 51) |
| | L3 | DIQMTQSPSSLSASVGDRVTITCKASENVVSYVSWY QQKPGKAPKLLIYGASNRYTGVPSRFIGSGSSTDFTL TISSLQPEDFATYYCGQSYSYPLTFGQGTKLEIK RTVAAPSVFIAPPSDEQLKSGTASVVCLLNNFYPREA KVQWKVDNALQSGNSQESVTEQDSKDSTYSLSSTLT LSKADYEKHKVYACEVTHQGLSSPVTKSFNRGEC (SEQ ID NO: 52) |

DNA sequences encoding and constituting each polypeptide chain were inserted into vectors (e.g., pcDNA3.1 vector disclosed in CN107001463A, pCHO1.0 vector disclosed in CN109422811A, etc.), respectively, to obtain expression vectors expressing the corresponding polypeptide chain. According to a mass ratio in equal proportion, the expression vectors encoding each polypeptide chain were co-transfected into ExpiCHO-S cells (manufacturer: Shanghai Institute of Pharmaceutical Industry, Cat No. 127200005) at a transfection expression volume of 0.1-1 L and a cell density of 6E+06 cells/mL. After transfection, the cells were placed in ExpiCHO^{™} medium (manufacturer: Thermo, Cat No. A2910001), and expressed and cultured at 32 °C with 5% CO₂ at 130 rpm. On day 10, the cell supernatant was collected, and protein A affinity chromatography was performed on the target protein using AKTA Pure 25 L system from GE and MabSelect SuRe LX packing. After elution, the concentration of the eluted protein was measured using NanoDrop Lite (Thermo Fisher Scientific). The purity of the eluted protein was measured by SEC-HPLC.

**Table 12. Expression amount and purity of anti-PDL1*CD47 bispecific antibodies**

| Sample | Purification method | Expression amount (mg/L) | HPLC-SEC purity (%) |
|---|---|---|---|
| MHL147-3322-IgG1-wt | Protein A affinity chromatography | 22 | 82.94 |
| MHL147-3322-IgG1-F118A | Protein A affinity chromatography | 38 | 92.85 |

The purification results of one-step protein A affinity chromatography showed that MHL147-3322-IgG1-F118A had higher expression amount and purity than MHL147-3322-IgG1-wt (Table 12), and the single-point mutation F118A (EU numbering) in the CL domain could improve the assembly and pairing process of the bispecific antibody in cells and reduce the formation of mispaired products, thereby improving the expression amount and purity of the bispecific antibody.

### 5.2. Thermal stability

The thermal stability of HZ14A9, HZ5G11, MHL147-3322-IgG1-wt, and MHL147-3322-IgG1-F118A was characterized by Nano DSF (Nano Temper). The concentration of the sample to be tested was adjusted to about 1.0 mg/mL, then a small amount of the sample was sucked using a silica capillary and placed in a Nano DSF sample suspension. The temperature was raised in a range of 20.0 °C to 95.0 °C at a constant speed of 1.0 °C/min. The optical signal change of the protein sample was collected, and the thermal stability of the protein was analyzed.

**Table 13. Thermal stability of anti-PDL1*CD47 bispecific antibodies**

| Sample | Melting temperature Tm1 (°C) |
|---|---|
| MHL147-3322-IgG1-wt | 66.4 |
| MHL147-3322-IgG1-F118A | 67 |
| HZ14A9 | 68.3 |
| HZ5G11 | 67 |

The Nano DSF experimental results showed that HZ14A9, HZ5G11, MHL147-3322-IgG1-wt, and MHL147-3322-IgG1-F118A all had good thermal stability, and the melting temperature Tm1 of all samples was higher than 65 °C (Table 13). The single-point mutation F118A (EU numbering) in the CL domain had no significant effect on the thermal stability of the bispecific antibodies.

### 5.3. Affinity

The affinity properties of the HZ5G11 monoclonal antibody, the HZ14A9 monoclonal antibody, MHL147-3322-IgG1-wt and MHL147-3322-IgG1-F118A bispecific antibodies were characterized by Biacore. The HZ5G11 monoclonal antibody, the HZ14A9 monoclonal antibody, MHL147-3322-IgG1-wt and MHL147-3322-IgG1-F118A bispecific antibodies were respectively immobilized onto a sensing chip by a capture method, and a human PDL1 (manufacturer: ACROBiosystems, Cat. No. PD1-H52H3) or human CD47 (manufacturer: ACROBiosystems, Cat. No. CD7-H5227) protein antigen solution with a certain concentration gradient was injected into the sample. Association/dissociation curves were acquired in real time by Biacore 8K Control Software 3.0, and data analysis was performed by Biacore Insight Evaluation Software 3.0. An association rate constant Ka, a dissociation rate constant Kd, and an equilibrium constant KD were obtained.

**Table 14. Binding affinity of anti-PDL1*CD47 bispecific antibodies to human CD47 antigen**

| Sample | ka (1/Ms) | kd (1/s) | KD (M) |
|---|---|---|---|
| HZ14A9 monoclonal antibody | 2.45E+06 | 1.43E-02 | 5.85E-09 |
| MHL147-3322-IgG1-F118A | 4.74E+06 | 2.49E-02 | 5.26E-09 |
| MHL147-3322-IgG1-wt | 3.75E+06 | 1.24E-02 | 3.31E-09 |

**Table 15. Binding affinity of anti-PDL1*CD47 bispecific antibodies to human PDL1 antigen**

| Sample | ka (1/Ms) | kd (1/s) | KD (M) |
|---|---|---|---|
| HZ5G11 monoclonal antibody | 1.51E+06 | 4.27E-04 | 2.82E-10 |
| MHL147-3322-IgG1-F118A | 1.58E+06 | 6.06E-04 | 3.84E-10 |
| MHL147-3322-IgG1-wt | 1.85E+06 | 5.62E-04 | 3.04E-10 |

The experimental results showed that the bispecific antibodies MHL147-3322-IgG1-wt and MHL147-3322-IgG1-F118A both exhibited good affinity to human PDL1 and CD47 proteins (Table 15 and Table 16).

### 5.4. Red blood cell agglutination experiment

Potential blood safety properties of MHL147-3322-IgG1-wt and MHL147-3322-IgG1-F118A were characterized by human red blood cell agglutination experiments. Red blood cells were washed thoroughly to remove plasma attached to the surface of the red blood cell membrane. The red blood cells were washed 3 times with isotonic diluent, centrifuged at 2000 rpm/min for 5 min for the first 2 times, and centrifuged at 2000 rpm/min for 10 min for the last time. The supernatant was then discarded. The red blood cells were prepared into a 2% cell suspension with PBS. 100 µL of the suspension was pipetted into a 96-well U-shaped plate, the plate was centrifuged at 1500 rpm/min for 5 min, and the supernatant was discarded. MHL147-3322-IgG1-wt, MHL147-3322-IgG1-F118A, and an isotype control hIgG1 sample were adjusted to 8 concentration gradients (0, 0.1, 0.2, 0.7, 2, 6, 17, and 50 µg/mL), an antibody diluent was added to the 96-well U-shaped plate, and the total reaction volume was 100 µL. The mixture was resuspended and mixed well, and then the plate was left to stand in an incubator at 37 °C for 2 h. The results were observed after incubation for 2 h. As shown in FIG. 10, MHL147-3322-IgG1-F118A showed weaker red blood cell agglutination ability than MHL147-3322-IgG1-wt at a bispecific antibody concentration ≥ 2 µg/mL, thereby suggesting that the F118A (EU numbering) mutation in the CL domain of the IgK constant region can effectively reduce the formation of mispaired products and improve the uniformity of bispecific antibodies.

## Claims

1. A bispecific antibody, comprising a first antigen-binding portion and a second antigen-binding portion that specifically bind to two different antigens or different epitopes of the same antigen, wherein the first antigen-binding portion comprises:
a first polypeptide comprising, from N-terminus to C-terminus, a first heavy chain variable domain and a first paired domain operably linked to the first heavy chain variable domain, and
a second polypeptide comprising, from N-terminus to C-terminus, a first light chain variable domain and a second paired domain operably linked to the first light chain variable domain,
wherein one of the first paired domain and the second paired domain comprises an amino acid sequence of engineered HLA-I α3, and the other paired domain comprises an amino acid sequence of engineered β2 microglobulin.

2. The bispecific antibody according to claim 1, wherein the first paired domain and the second paired domain can form a dimer, at least one non-natural interchain bond can be formed between the first paired domain and the second paired domain, and the non-natural interchain bond can stabilize the dimer.

3. The bispecific antibody according to claim 1 or 2, wherein the first paired domain comprises an amino acid sequence of the engineered HLA-I α3, and the second paired domain comprises an amino acid sequence of the engineered β2 microglobulin; or
the first paired domain comprises an amino acid sequence of the engineered β2 microglobulin, and the second paired domain comprises an amino acid sequence of the engineered HLA-I α3.

4. The bispecific antibody according to any one of claims 1 to 3, wherein the amino acid sequence of the engineered HLA-I α3 has at least 85%, 86%, 87%, 88%, 89%, 90%, 91%, 92%, 93%, 94%, 95%, 96%, 97%, 98%, 99%, or 100% identity to a sequence set forth in SEQ ID NO: 1, and the amino acid sequence of the engineered β2 microglobulin has at least 85%, 86%, 87%, 88%, 89%, 90%, 91%, 92%, 93%, 94%, 95%, 96%, 97%, 98%, 99%, or 100% identity to a sequence set forth in SEQ ID NO: 2.

5. The bispecific antibody according to any one of claims 1 to 4, wherein the engineered HLA-I α3 comprises an amino acid sequence having an amino acid substitution in the sequence set forth in SEQ ID NO: 1, and the engineered β2 microglobulin comprises an amino acid sequence having an amino acid substitution in the sequence set forth in SEQ ID NO: 2.

6. The bispecific antibody according to claim 5, wherein the amino acid substitutions in both the engineered HLA-I α3 and the engineered β2 microglobulin comprise cysteine residue substitutions that occur at a contact interface between the two and can form a disulfide bond with each other;
the cysteine residue substitution is selected from one or more pairs in the following group:
(1) R60C in SEQ ID NO: 1 and Y26C in SEQ ID NO: 2;
(2) A62C in SEQ ID NO: 1 and R12C in SEQ ID NO: 2; and
(3) G63C in SEQ ID NO: 1 and Y67C in SEQ ID NO: 2.

7. The bispecific antibody according to any one of claims 5 to 6, wherein the amino acid substitutions in the engineered HLA-I α3 or/and the engineered β2 microglobulin comprise amino acid substitutions that increase an isoelectric point of the dimer formed by the paired domains or the bispecific antibody,
wherein the amino acid substitutions that increase the isoelectric point comprise a substitution in the engineered HLA-I α3 with a positively charged amino acid at one or more positions of E3, D22, E48, D53, E90, and E101 in the sequence set forth in SEQ ID NO: 1; or/and
the amino acid substitutions that increase the isoelectric point comprise a substitution in the engineered β2 microglobulin with a positively charged amino acid at one or more positions of E74, E47, E69, D34, E16, D53, E44, E50, and E36 in the sequence set forth in SEQ ID NO: 2; preferably,
the amino acid substitutions that increase the isoelectric point comprise: amino acid substitutions D22R, E48K, and D53K comprised in the sequence set forth in SEQ ID NO: 1 for the engineered HLA-I α3, and an amino acid substitution E69R comprised in the sequence set forth in SEQ ID NO: 2 for the engineered β2 microglobulin.

8. The bispecific antibody according to any one of claims 1 to 7, wherein the engineered HLA-I α3 comprises an amino acid sequence set forth in SEQ ID NO: 3, and the engineered β2 microglobulin comprises an amino acid sequence set forth in SEQ ID NO: 4; or
the engineered HLA-I α3 comprises an amino acid sequence set forth in SEQ ID NO: 3, and the engineered β2 microglobulin comprises an amino acid sequence set forth in SEQ ID NO: 5.

9. The bispecific antibody according to any one of claims 1 to 8, wherein the second antigen-binding portion comprises a Fab; preferably,
an amino acid in a CL domain of the Fab is substituted with alanine at position F118 according to an EU numbering.

10. The bispecific antibody according to any one of claims 1 to 8, wherein the bispecific antibody is bivalent.

11. The bispecific antibody according to any one of claims 1 to 8, further comprising a third antigen-binding portion, wherein the third antigen-binding portion binds to the same antigen epitope as the second antigen-binding portion; or
the third antigen-binding portion binds to the same antigen epitope as the first antigen-binding portion.

12. The bispecific antibody according to claim 10, wherein the bispecific antibody is trivalent.

13. The bispecific antibody according to any one of claims 1 to 10, further comprising an Fc domain composed of two Fc polypeptides capable of stable association.

14. The bispecific antibody according to claim 13, wherein the first antigen-binding portion is operably linked at its C-terminus to N-terminus of one of the Fc polypeptides, and the second antigen-binding portion is operably linked at its C-terminus to N-terminus of the other Fc polypeptide; or
the first polypeptide of the first antigen-binding portion is operably linked at its C-terminus to N-terminus of one of the Fc polypeptides, and the second antigen-binding portion comprises a Fab and a Fab heavy chain of the second antigen-binding portion is operably linked at its C-terminus to N-terminus of the other Fc polypeptide.

15. The bispecific antibody according to any one of claims 11 to 12, further comprising an Fc domain composed of two Fc polypeptides capable of stable association.

16. The bispecific antibody according to claim 15, wherein the first antigen-binding portion is operably linked at its C-terminus to N-terminus of one of the Fc polypeptides, the second antigen-binding portion is operably linked at its C-terminus to N-terminus of the other Fc polypeptide, and the third antigen-binding portion is operably linked at its C-terminus to N-terminus of the first antigen-binding portion or N-terminus of the second antigen-binding portion; or
the third antigen-binding portion is operably linked at its C-terminus to N-terminus of one of the Fc polypeptides, the first antigen-binding portion is operably linked at its C-terminus to N-terminus of the third antigen-binding portion, and the second antigen-binding portion is operably linked at its C-terminus to N-terminus of the other Fc polypeptide; or
the third antigen-binding portion is operably linked at its C-terminus to N-terminus of one of the Fc polypeptides, the second antigen-binding portion is operably linked at its C-terminus to N-terminus of the third antigen-binding portion, and the first antigen-binding portion is operably linked at its C-terminus to N-terminus of the other Fc polypeptide.

17. A method of treating a disease in a subject in need thereof, comprising administering to the subject a therapeutically effective amount of the bispecific antibody according to any one of claims 1 to 16, wherein preferably, the disease comprises leukemia, lymphoma, myeloma, ovarian cancer, breast cancer, endometrial cancer, colon cancer, rectal cancer, kidney cancer, bladder cancer, urothelial cancer, lung cancer, bronchial cancer, bone cancer, prostate cancer, pancreatic cancer, gastric cancer, hepatocellular carcinoma, gallbladder cancer, bile duct cancer, esophageal cancer, renal cell cancer, thyroid cancer, head and neck cancer, testicular cancer, endocrine adenocarcinoma, adrenal cancer, pituitary gland cancer, skin cancer, soft tissue cancer, vascular cancer, brain cancer, nerve cancer, eye cancer, meningeal cancer, oropharyngeal cancer, hypopharynx cancer, cervical cancer, uterine cancer, glioblastoma, medulloblastoma, astrocytoma, glioma, meningioma, gastrinoma, neuroblastoma, melanoma, myelodysplastic syndrome, or sarcoma.
